# EUROPEAN PATENT APPLICATION

(11) **EP 4 273 170 A1**
(43) Date of publication of application: **08.11.2023**
(21) Application number: 23745373.3
(22) Date of filing: 14.02.2023
(51) Int. Cl.: C07K 16/28, A61K 39/395, A61P 35/00

(54) **PHARMACEUTICAL COMPOSITION COMPRISING ANTI-TIGIT ANTIBODY AND ANTI-PD-1-ANTI-VEGFA BISPECIFIC ANTIBODY, AND USE**

(30) Priority: 14.02.2022 CN 202210132762
(71) Applicant: Akeso Biopharma, Inc., Zhongshan, Guangdong 528437 (CN)
(72) Inventor: XIA, Yu, Zhongshan, Guangdong 528437 (CN); WANG, Zhongmin, Zhongshan, Guangdong 528437 (CN); LI, Baiyong, Zhongshan, Guangdong 528437 (CN)
(74) Representative: Cooley (UK) LLP
(86) International application number: PCT/CN2023/075862
(87) International publication number: WO 2023/151693

(57) **Abstract**

The present invention relates to the field of pharmaceuticals, particularly to an anti-TIGIT antibody, a pharmaceutical composition, and use thereof, and more particularly to use in combination with an anti-PD-1/anti-VEGFA antibody in preventing or treating a tumor. Specifically, the present invention relates to an anti-TIGIT antibody or an antigen-binding fragment thereof, wherein the antibody comprises a heavy chain variable region comprising HCDR1-HCDR3 having amino acid sequences set forth in SEQ ID NOs: 3-5, respectively; and the antibody comprises a light chain variable region comprising LCDR1-LCDR3 having amino acid sequences set forth in SEQ ID NOs: 8-10, respectively. The antibody of the present invention can effectively bind to TIGIT and has the potential for use in tumor prevention and treatment.

## Description

### TECHNICAL FIELD

The present invention belongs to the field of medicine, and relates to an anti-TIGIT antibody, and a pharmaceutical composition and the use thereof. More particularly, the present invention relates to an anti-TIGIT monoclonal antibody.

### BACKGROUND

TIGIT (T cell Ig and ITIM domain, also known as WUCAM, Vstm3, or VSIG9) is a member of the poliovirus receptor (PVR)/Nectin family. TIGIT consists of an extracellular immunoglobulin variable region (IgV) domain, a type I transmembrane domain, and an intracellular domain with a classical immunoreceptor tyrosine-based inhibitory motif (ITIM) and an immunoglobulin tail tyrosine (ITT) motif. TIGIT is overexpressed in lymphocytes, particularly effector and regulatory CD4+ T cells, follicular helper CD4+ T cells and effector CD8+ T cells, as well as natural killer (NK) cells (Yu X, Harden K, Gonzalez L C, et al., The surface protein TIGIT suppresses T cell activation by promoting the generation of mature immunoregulatory dendritic cells[J]. Nature Immunology, 2009, 10(1): 48).

CD155 (also known as PVR, Necl5 or Tage4), CD112 (also known as PVRL2/nectin 2) and CD113 (also known as PVRL3) are ligands to which TIGIT binds (Martinet L, Smyth M J., Balancing natural killer cell activation through paired receptors[J]. Nature Reviews Immunology, 2015, 15(4): 243-254), wherein CD155 is a high-affinity ligand for TIGIT. In NK cells, the binding of TIGIT to ligand CD155 or CD112 can inhibit the killing effect of NK cells on TIGIT-overexpressing cells (Stanietsky N, Simic H, Arapovic J, et al., The interaction of TIGIT with PVR and PVRL2 inhibits human NK cell cytotoxicity[J]. Proceedings of the National Academy of Sciences, 2009, 106(42): 17858-17863). It was also reported that when PD-1 and TIGIT are blocked simultaneously, the killing effect of CD8+ T cells can be enhanced (Johnston R J, Comps-Agrar L, Hackney J, et al., The immunoreceptor TIGIT regulates antitumor and antiviral CD8+ T cell effector function[J]. Cancer Cell, 2014, 26(6): 923-937). In recent studies, TIGIT was found to be an immune checkpoint of NK cells and capable of causing NK cell depletion as an inhibitory receptor in the process of tumor progression; it was also demonstrated that anti-TIGIT monoclonal antibody can reverse NK cell depletion and be used as immunotherapy for various tumors (Zhang Q, Bi J, Zheng X, et al., Blockade of the checkpoint receptor TIGIT prevents NK cell exhaustion and elicits potent anti-tumor immunity[J]. Nature immunology, 2018, 19(7): 723-732).

In addition, it was reported that TIGIT blockers alone or in combination with PD-1 blockers plus CD96 blockers could significantly reduce the growth of B16 melanoma in wild-type and CD155-/- mouse models (Li X-Y, Das I, Lepletier A, et al., CD155 loss enhances tumor suppression via combined host and tumor-intrinsic mechanisms. J Clin Invest, 2018, 128:2613-25). CD112R blockers alone or in combination with TIGIT blockers and/or PD-1 blockers could increase cytokine production ability of TILs in ovarian cancer, endometrial cancer and lung tumor (Whelan S, Ophir E, Kotturi MF, et al., PVRIG and PVRL2 Are Induced in Cancer and Inhibit CD8+ T-cell function. Cancer Immunol Res, 2019, 7:257-68). Anti-TIGIT antibody medicaments, as new immune checkpoint antibody medicaments, have promising utility in a variety of applications, and can be used for immunotherapy of tumors. Tiragolumab developed by Roche is now in phase 3 clinical trials, and it was reported that the combination of the TIGIT monoclonal antibody Tiragolumab and the PD-L1 medicament Tecentriq (atezolizumab) as first-line therapy was well tolerated in patients with PD-L1-positive metastatic non-small cell lung cancer (NSCLC) in phase 2 clinical trials and had a significant effect—a 43% reduction in the risk of disease progression (Exit C. Roche to present first clinical data on novel anti-TIGIT cancer immunotherapy tiragolumab at ASCO[J]). Existing clinical records indicate that TIGIT is an important target for treating non-small cell lung cancer, small cell lung cancer, breast cancer, ovarian cancer, colorectal cancer, melanoma, pancreatic cancer, cervical tumor, multiple myeloma, non-Hodgkin's lymphoma, B-lymphoma, and plasma cell cancer. However, the affinity of existing anti-human TIGIT antibody medicaments is low; there is still a need for anti-TIGIT antibodies with high affinity.

Therefore, the development of antibody medicaments with a high affinity for TIGIT, higher efficacy, and fewer toxic side effects for treating autoimmune diseases is of great significance.

The transmembrane receptor PD-1 (programmed cell death protein-1) is a member of the CD28 family, and is expressed in activated T cells, B cells, and myeloid cells. Both ligands of PD-1, PDL1 (programmed cell death 1 ligand 1, or PD-L1) and PDL2 (programmed cell death 1 ligand 2, or PD-L2), are members of the B7 superfamily, wherein PDL1 is expressed in a variety of cells including T cells, B cells, endothelial cells, and epithelial cells, and PDL2 is expressed only in antigen-presenting cells such as dendritic cells and macrophages.

The PD-1/PDL1 signaling pathway plays an important role in regulating immune tolerance, microbial infection, and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, activate the tumor cell killing process, and further inhibit local tumor growth (Julie R et al., 2012, N Engl J Med., 366:2455-2465). In addition, tumors with high PDL1 expression are associated with cancers that are difficult to detect (Hamanishi et al., 2007, Proc. Natl. Acad. Sci. USA, 104:3360-5). An effective method is administering an anti-PD-1 antibody to modulate the expression of PD-1. Due to the broad anti-tumor prospects and surprising efficacy of PD-1 antibodies, it is widely accepted in the industry that antibodies targeting the PD-1 pathway will bring about breakthroughs in the treatment of various tumors, for example, non-small cell lung cancer, renal cell carcinoma, ovarian cancer, melanoma (Hornet M. B., Parisi G., et al., 2015, Semin Oncol., 42(3):466-473), leukemia and anemia (Held SA, Heine A, et al., 2013, Curr Cancer Drug Targets., 13(7):768-74).

Vascular endothelial growth factor (VEGF) is a growth factor that can promote the division and proliferation of endothelial cells, promote the formation of new blood vessels and improve blood vessel permeability. It binds to vascular endothelial growth factor receptors on the cell surface and plays a role by activating tyrosine kinase signal transduction pathways. In tumor tissues, tumor cells, and macrophages and mast cells invading tumors can secrete a high level of VEGF, stimulate tumor vascular endothelial cells in a paracrine form, promote proliferation and migration of endothelial cells, induce angiogenesis, promote continuous growth of tumors, improve vascular permeability, cause fibrin deposition in surrounding tissues, and promote infiltration of mononuclear cells, fibroblasts and endothelial cells, which facilitates the formation of tumor stroma and entry of tumor cells into new blood vessels and promotes tumor metastasis. Therefore, inhibiting tumor angiogenesis is considered as one of the most promising tumor treatment methods at present. The VEGF family includes VEGFA, VEGFB, VEGFC, VEGFD, and PIGF. Vascular endothelial growth factor receptors (VEGFRs) include VEGFR1 (also known as Flt1), VEGFR2 (also known as KDR or Flk1), VEGFR3 (also known as Flt4) and Neuropilin-1 (NRP-1). The first three receptors are members of the tyrosine kinase superfamily, and are of similar structures composed of an extramembrane region, a transmembrane segment and an intramembrane region, where the extramembrane region is composed of an immunoglobulin-like domain, and the intramembrane region is a tyrosine kinase region. VEGFR1 and VEGFR2 are mainly found on the surface of vascular endothelial cells, and VEGFR3 is mainly found on the surface of lymphatic endothelial cells.

Molecules of the VEGF family have different affinities for these receptors. VEGF A mainly acts in combination with VEGFR1, VEGFR2 and NRP-1. VEGFR1 is the first identified receptor, and has a higher affinity for VEGF A than VEGFR2 under normal physiological conditions but a lower tyrosinase activity in the intracellular segment than VEGFR2 (Ma Li, Chinese Journal of Birth Health and Heredity, 24(5), 2016:146-148).

VEGFR2 is the primary regulator of angiogenesis and vascular engineering, and has a much higher tyrosine kinase activity than VEGFR1. VEGFR2, after binding to ligand VEGFA, mediates the proliferation, differentiation and other progresses of vascular endothelial cells, as well as the formation process of blood vessels and the permeability of blood vessels (Roskoski R Jr. et al., Crit Rev Oncol Hematol, 62(3), 2007:179-213). VEGFA, after binding to VEGFR2, mediates the transcription and expression of related intracellular protein genes through the downstream PLC-γ-PKC-Raf-MEK-MAPK signaling pathway, and thus promotes the proliferation of vascular endothelial cells (Takahashi T et al., Oncogene, 18(13), 1999:2221-2230).

VEGFR3 is one of the tyrosine kinase family members, and is mainly expressed in embryonic vascular endothelial cells and mature lymphatic endothelial cells, and VEGFC and VEGFD bind to VEGFR3 to stimulate proliferation and migration of lymphatic endothelial cells and promote neogenesis of lymphatic vessels; NRP-1 is a non-tyrosine kinase transmembrane protein, and is incapable of independently transducing biological signals but able to mediate signaling only after forming a complex with a VEGF tyrosine kinase receptor. (Ma Li, Chinese Journal of Birth Health and Heredity, 24(5), 2016:146-148).

VEGF A and VEGFR2 are mainly involved in regulation of angiogenesis, where before and after the binding of VEGF A to VEGFR2, a cascade reaction of numerous intermediate signals in upstream and downstream pathways is formed, and finally the physiological functions are changed by proliferation, survival, migration, permeability increase, infiltration to peripheral tissues and other patterns of endothelial cells (Dong Hongchao et al., Sep. 2014, Journal of Modern Oncology, 22(9): 2231-3).

Currently, there are several humanized monoclonal antibodies targeting human VEGF, particularly VEGFA, such as bevacizumab, which has been approved by the U.S. Food and Drug Administration for the treatment of various tumors such as non-small cell lung cancer, renal cell carcinoma, cervical cancer, and metastatic colorectal cancer in succession during 2004.

In summary, developing a treatment or combination therapy with higher efficacy is of great meaning.

### SUMMARY

After intensive studies and creative efforts, the inventors used mammalian cell expression systems to express recombinant human TIGIT as an antigen to immunize mice, and obtained hybridoma cells by fusion of mouse spleen cells and myeloma cells. The inventor obtained a hybridoma cell line LT019 (deposited under CCTCC NO. C2020208) by screening a large number of samples.

The inventors surprisingly found that the hybridoma cell line LT019 can secrete a specific monoclonal antibody (designated 26B12) specifically binding to human TIGIT, and the monoclonal antibody can effectively bind to TIGIT, reduce the inhibitory effect of TIGIT on immune cells, promote the activity of T cells, reverse NK cell exhaustion, and enhance the killing effect of immune cells on a tumor. Further, the inventors have creatively prepared humanized anti-human TIGIT antibodies (designated 26B12H1L1, 26B12H4L1, 26B12H2L2, 26B12H3L2, 26B12H2L3, 26B12H3L3, 26B12H1L4, and 26B 12H4L4).

The inventors also surprisingly found that antibodies 26B12H1L1, 26B12H4L1, 26B12H2L2, 26B12H3L2, 26B12H2L3, 26B12H3L3, 26B12H1L4, and 26B12H4L4 of the present invention have binding activity to TIGIT and have strong affinity; 26B12H1L1, 26B12H4L1, 26B12H2L2, 26B12H3L2, 26B12H2L3, 26B12H3L3, 26B12H1L4, and 26B12H4L4 can effectively reduce the activity of TIGIT.

Furthermore, the inventors found that the anti-TIGIT antibodies in combination with anti-PD-1/anti-VEGFA bispecific antibodies are able to effectively prevent and treat tumors.

The antibodies of the present invention have potentials for use in treating and/or preventing of diseases such as tumors (e.g., non-small cell lung cancer, small cell lung cancer, breast cancer, ovarian cancer, colorectal cancer, melanoma, pancreatic cancer, cervical cancer, multiple myeloma, non-Hodgkin's lymphoma, and plasma cell cancer). The present invention is detailed below.

One aspect of the present invention relates to an anti-TIGIT antibody or an antigen-binding fragment thereof, wherein
the anti-TIGIT antibody comprises HCDR1-HCDR3 comprised in a heavy chain variable region set forth in SEQ ID NO: 1 and LCDR1-LCDR3 comprised in a light chain variable region set forth in SEQ ID NO: 6,
preferably, according to the IMGT numbering system, the antibody comprises a heavy chain variable region comprising HCDR1-HCDR3 having amino acid sequences set forth in SEQ ID NOs: 3-5, respectively, and a light chain variable region of the antibody comprises LCDR1-LCDR3 with amino acid sequences set forth in SEQ ID NOs: 8-10, respectively.

In one or more embodiments of the present invention, the heavy chain variable region of the antibody has an amino acid sequence selected from SEQ ID NO: 1, SEQ ID NO: 11, SEQ ID NO: 13, SEQ ID NO: 15, and SEQ ID NO: 17; and
the light chain variable region of the antibody has an amino acid sequence selected from SEQ ID NO: 6, SEQ ID NO: 19, SEQ ID NO: 21, SEQ ID NO: 23, and SEQ ID NO: 25.

In one or more embodiments of the present invention, the heavy chain variable region of the antibody has an amino acid sequence set forth in SEQ ID NO: 1, and the light chain variable region of the antibody has an amino acid sequence set forth in SEQ ID NO: 6;
the heavy chain variable region of the antibody has an amino acid sequence set forth in SEQ ID NO: 11, and the light chain variable region of the antibody has an amino acid sequence set forth in SEQ ID NO: 19;
the heavy chain variable region of the antibody has an amino acid sequence set forth in SEQ ID NO: 17, and the light chain variable region of the antibody has an amino acid sequence set forth in SEQ ID NO: 19;
the heavy chain variable region of the antibody has an amino acid sequence set forth in SEQ ID NO: 13, and the light chain variable region of the antibody has an amino acid sequence set forth in SEQ ID NO: 21;
the heavy chain variable region of the antibody has an amino acid sequence set forth in SEQ ID NO: 13, and the light chain variable region of the antibody has an amino acid sequence set forth in SEQ ID NO: 23;
the heavy chain variable region of the antibody has an amino acid sequence set forth in SEQ ID NO: 15, and the light chain variable region of the antibody has an amino acid sequence set forth in SEQ ID NO: 21;
the heavy chain variable region of the antibody has an amino acid sequence set forth in SEQ ID NO: 15, and the light chain variable region of the antibody has an amino acid sequence set forth in SEQ ID NO: 23;
the heavy chain variable region of the antibody has an amino acid sequence set forth in SEQ ID NO: 11, and the light chain variable region of the antibody has an amino acid sequence set forth in SEQ ID NO: 25; or
the heavy chain variable region of the antibody has an amino acid sequence set forth in SEQ ID NO: 17, and the light chain variable region of the antibody has an amino acid sequence set forth in SEQ ID NO: 25.

In one or more embodiments of the present invention, the antibody comprises a non-CDR region derived from a species other than murine, such as from a human antibody.

In one or more embodiments of the present invention, the antibody comprises a heavy chain constant region that is an Ig gamma-1 chain C region (e.g., NCBI ACCESSION: P01857), and a light chain constant region that is an Ig kappa chain C region (e.g., NCBI ACCESSION: P01834).

In one or more embodiments of the present invention, the anti-TIGIT antibody or the antigen-binding fragment thereof is selected from Fab, Fab', F(ab')₂, Fd, Fv, dAb, a complementarity determining region fragment, a single chain antibody, a humanized antibody, a chimeric antibody, and a diabody.

In one or more embodiments of the present invention, for the anti-TIGIT antibody or the antigen-binding fragment thereof, the antibody binds to TIGIT-mFc with a K_{D} less than 4E-10 or less than 4E-11; preferably, the K_{D} is measured by a Fortebio molecular interaction instrument.

In one or more embodiments of the present invention, for the anti-TIGIT antibody or the antigen-binding fragment thereof, the antibody binds to TIGIT with an EC₅₀ less than 1.5 nM, less than 1.2 nM, or less than 1 nM; preferably, the EC₅₀ is measured by a flow cytometer.

In some embodiments of the present invention, the anti-TIGIT antibody is a monoclonal antibody.

In some embodiments of the present invention, the anti-TIGIT antibody is a humanized antibody, a chimeric antibody, or a multispecific antibody (e.g., a bispecific antibody).

In some embodiments of the present invention, the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody, and a bispecific antibody.

In one or more embodiments of the present invention, for the anti-TIGIT antibody or the antigen-binding fragment thereof, the antibody is an antibody produced by hybridoma cell line LT019 deposited at China Center for Type Culture Collection (CCTCC; Wuhan, China, postal code: 430072) under CCTCC NO. C2020208.

Another aspect of the present invention relates to an isolated nucleic acid molecule encoding the anti-TIGIT antibody or the antigen-binding fragment thereof according to any aspect of the present invention.

Yet another aspect of the present invention relates to a vector comprising the isolated nucleic acid molecule of the present invention.

Yet another aspect of the present invention relates to a host cell comprising the isolated nucleic acid molecule or the vector of the present invention.

Yet another aspect of the present invention relates to a hybridoma cell line LT019 deposited at China Center for Type Culture Collection (CCTCC) under CCTCC NO. C2020208.

Yet another aspect of the present invention relates to a conjugate comprising an antibody and a conjugated moiety, wherein the antibody is the anti-TIGIT antibody or the antigen-binding fragment thereof according to any aspect of the present invention, and the conjugated moiety is a detectable label; preferably, the conjugated moiety is a radioisotope, a fluorescent substance, a luminescent substance, a colored substance, or an enzyme.

Yet another aspect of the present invention relates to a kit comprising the anti-TIGIT antibody or the antigen-binding fragment thereof according to any aspect of the present invention or comprising the conjugate of the present invention;
wherein preferably, the kit further comprises a second antibody specifically recognizing the antibody; optionally, the second antibody further comprises a detectable label, for example, a radioisotope, a fluorescent substance, a luminescent substance, a colored substance, or an enzyme.

Yet another aspect of the present invention relates to use of the antibody according to any aspect of the present invention or the conjugate of the present invention in preparing a kit for detecting the presence or level of TIGIT in a sample.

Yet another aspect of the present invention relates to a pharmaceutical composition comprising the anti-TIGIT antibody or the antigen-binding fragment thereof according to any aspect of the present invention or the conjugate of the present invention; optionally, the pharmaceutical composition further comprises a pharmaceutically acceptable carrier and/or excipient.

In one or more embodiments of the present invention, the pharmaceutical composition further comprises one or more anti-PD-1 antibodies or anti-VEGF antibodies.

In one or more embodiments of the present invention, for the pharmaceutical composition, the anti-TIGIT antibody or the antigen-binding fragment thereof and the anti-PD-1 antibody or the anti-VEGF antibody are present in a mass ratio, on antibody basis, of 1:5-5:1, for example, 1:5, 1:4, 1:3, 1:2, 1:1, 2:1, 3:1, 4:1, or 5:1.

Yet another aspect of the present invention relates to a combination product (e.g., a kit) comprising a first product and a second product in separate packages, wherein, the first product comprises the anti-TIGIT antibody or the antigen-binding fragment thereof according to any aspect of the present invention, the conjugate of the present invention, or the pharmaceutical composition according to any aspect of the present invention;
the second product comprises at least one anti-PD-1 antibody or one anti-VEGF antibody, e.g., an anti-PD-1/anti-VEGFA bispecific antibody;
preferably, the combination product further comprises a third product in a separate package comprising one or more chemotherapeutics,
preferably, the first product and the second product further independently comprise one or more pharmaceutically acceptable excipients;
preferably, the combination product further comprises a package insert.

In one or more embodiments of the present invention, for the combination product, the anti-TIGIT antibody or the antigen-binding fragment thereof and the anti-PD-1 antibody or the anti-VEGF antibody are present in a mass ratio, on antibody basis, of 1:5-5:1, for example, 1:5, 1:4, 1:3, 1:2, 1:1, 2:1, 3:1, 4:1, or 5:1.

In one or more embodiments of the present invention, the anti-PD-1 antibody or the anti-VEGF antibody is an anti-PD-1/anti-VEGFA bispecific antibody.

Yet another aspect of the present invention relates to use of the antibody according to any aspect of the present invention, the conjugate of the present invention, the pharmaceutical composition according to any aspect of the present invention, or the combination according to any aspect of the present invention in preparing a medicament for treating and/or preventing a tumor; preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, and plasma cell cancer.

The antibody according to any aspect of the present invention, the conjugate of the present invention, the pharmaceutical composition according to any aspect of the present invention, or the combination according to any aspect of the present invention is for use in treating and/or preventing a tumor; preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, and plasma cell cancer.

Yet another aspect of the present invention relates to a method for treating and/or preventing a tumor, comprising administering to a subject in need an effective amount of the antibody according to any aspect of the present invention, the conjugate of the present invention, the pharmaceutical composition according to any aspect of the present invention, or the combination according to any aspect of the present invention; preferably, the tumor is selected from one or more of non-small cell lung cancer, small cell lung cancer, breast cancer, ovarian cancer, colorectal cancer, melanoma, pancreatic cancer, cervical cancer, multiple myeloma, non-Hodgkin's lymphoma, and plasma cell cancer.

The present invention relates to a method for preventing and/or treating a tumor (particularly a malignant tumor), comprising administering to a subject a therapeutically effective amount of the anti-TIGIT antibody in combination with the anti-PD-1/anti-VEGFA bispecific antibody, and more preferably further in combination with one or more therapeutics for treating a tumor (preferably the therapeutic is a chemotherapeutic or a growth inhibitor, a targeted therapeutic, an antibody-drug conjugate, a T cell expressing a chimeric antigen receptor, an antibody or an antigen-binding fragment thereof, an angiogenesis inhibitor, an antineoplastic, a cancer vaccine, an adjuvant and a combination thereof, an alkylating agent, an antimetabolite, an antibiotic, a plant-based and/or hormonal drug, preferably cyclophosphamide, pemetrexed, a platinum-based drug such as cisplatin, carboplatin, oxaliplatin, adriamycin, paclitaxel, vinca alkaloid, tamoxifen, megestrol, goserelin, asparaginase, and/or a fluorouracil antineoplastic); preferably, the anti-TIGIT antibody, the anti-PD-1/anti-VEGFA bispecific antibody, and the anti-tumor chemotherapeutic are administered either simultaneously or sequentially.

In one or more embodiments of the present invention, the chemotherapeutic or the growth inhibitor is selected from an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxic agent or an anti-tumor antibiotic, a proteasome inhibitor, an anti-microtubule agent, an EGFR antagonist, a VEGF antagonist, an angiopoietin 2 antagonist, a retinoid, a tyrosine kinase inhibitor, a histone deacetylase inhibitor, and a combination thereof.

In one or more embodiments of the present invention, the targeted therapeutic is selected from a B-raf inhibitor, an MEK inhibitor, a K-ras inhibitor, a c-Met inhibitor, an Alk inhibitor, a phosphatidylinositol 3-kinase inhibitor, an Akt inhibitor, an mTOR inhibitor, a VEGF inhibitor, a diphosphatidylglycol 3-kinase/mTOR inhibitor, and a combination thereof.

In one or more embodiments of the present invention, the antibody-drug conjugate comprises a drug selected from the group consisting of: maytansine, monomethyl auristatin E, calicheamicin, esperamicin, and a radioisotope chelating agent.

In one or more embodiments of the present invention, the tumor is selected from one or more of the following:
cervical cancer (e.g., metastatic cervical cancer), endometrial cancer, lung cancer such as small cell lung cancer and non-small cell lung cancer (e.g., squamous non-small cell lung cancer or non-squamous non-small cell lung cancer), throat cancer, esophageal cancer, esophageal squamous cancer, thyroid cancer, mesothelioma, gastric cancer (e.g., advanced gastric cancer, gastrointestinal cancer, gastric adenocarcinoma, or gastroesophageal junction adenocarcinoma), liver cancer (e.g., hepatocellular carcinoma), intestinal cancer, rectal cancer, colon cancer, colorectal cancer, cholangiocarcinoma, hepatobiliary cancer, biliary tract cancer, cholangiocarcinoma, pancreatic cancer, pancreatic cancer, renal cancer (e.g., renal cell carcinoma), ovarian cancer (e.g., advanced ovarian cancer), fallopian tube cancer, peritoneal cancer, glioma (e.g., neuroglioma and recurrent glioma), skin cancer, melanoma, leukemia (e.g., acute myeloid leukemia), lymphoma (e.g., Hodgkin's lymphoma and non-Hodgkin's lymphoma), plasma cell cancer, bone cancer, sarcoma, osteosarcoma, chondrosarcoma, neuroblastoma, myeloma (e.g., multiple myeloma), large cell neuroendocrine cancer, urothelial carcinoma (e.g., upper urothelial carcinoma or bladder cancer), prostate cancer, testicular cancer, peripheral T-cell lymphoma, nasopharyngeal cancer, high microsatellite instability (MSI-H) or mismatch repair deficient (dMMR) solid tumors, head and neck cancer, brain cancer (e.g., aggressive brain cancer, such as glioblastoma), squamous cell carcinoma, basal cell carcinoma, adenoma, breast cancer (e.g., triple-negative breast cancer), thymus cancer, ileocecal adenocarcinoma, ampullate adenocarcinoma, mucinous or serous cystadenocarcinoma, leiomyosarcoma, rhabdomyosarcoma, chorioepithelioma, malignant hydatidiform mole, malignant sertoli cell-stromal cell tumor, malignant granulocytoma, dysgerminoma, glioblastoma, mycosis, Merkel cell carcinoma, and other hematologic malignancies.

In one or more embodiments of the present invention, the anti-PD-1 bispecific antibody comprises:
a first protein functional region targeting PD-1, and
a second protein functional region targeting VEGFA;
wherein the first protein functional region is an immunoglobulin, and the second protein functional region is a single chain antibody; or, the first protein functional region is a single chain antibody, and the second protein functional region is an immunoglobulin;
wherein,
the immunoglobulin comprises a heavy chain variable region comprising HCDR1-HCDR3 comprised in a heavy chain variable region having an amino acid sequence set forth in SEQ ID NO: 31 (preferably, HCDR1-HCDR3 set forth in SEQ ID NOs: 35-37, respectively, according to the IMGT numbering system), and a light chain variable region comprising LCDR1-LCDR3 comprised in a light chain variable region having an amino acid sequence set forth in SEQ ID NO: 33 (preferably LCDR1-LCDR3 set forth in SEQ ID NOs: 38-40, respectively, according to the IMGT numbering system);
the single chain antibody comprises a heavy chain variable region comprising HCDR1-HCDR3 comprised in a heavy chain variable region having an amino acid sequence set forth in SEQ ID NO: 41 (preferably, HCDR1-HCDR3 set forth in SEQ ID NOs: 45-47, respectively, according to the IMGT numbering system), and a light chain variable region having an amino acid sequence 43 (preferably LCDR1-LCDR3 set forth in SEQ ID NOs: 48-50, respectively, according to the IMGT numbering system);
   or,
the immunoglobulin comprises a heavy chain variable region comprising HCDR1-HCDR3 comprised in a heavy chain variable region having an amino acid sequence set forth in SEQ ID NO: 41 (preferably, HCDR1-HCDR3 set forth in SEQ ID NOs: 45-47, respectively, according to the IMGT numbering system), and a light chain variable region comprising LCDR1-LCDR3 comprised in a light chain variable region having an amino acid sequence set forth in SEQ ID NO: 43 (preferably LCDR1-LCDR3 set forth in SEQ ID NOs: 48-50, respectively, according to the IMGT numbering system);
the single chain antibody comprises a heavy chain variable region comprising HCDR1-HCDR3 comprised in a heavy chain variable region having an amino acid sequence set forth in SEQ ID NO: 31 (preferably, HCDR1-HCDR3 set forth in SEQ ID NOs: 35-37, respectively, according to the IMGT numbering system), and a light chain variable region comprising LCDR1-LCDR3 comprised in a light chain variable region having an amino acid sequence set forth in SEQ ID NO: 33 (preferably LCDR1-LCDR3 set forth in SEQ ID NOs: 38-40, respectively, according to the IMGT numbering system);
the immunoglobulin is of human IgG1 subtype.

In one or more embodiments of the present invention, for the bispecific antibody, according to the EU numbering system, the heavy chain constant region of the immunoglobulin has the following mutations:
L234A and L235A; or
L234A and G237A; or
L235A and G237A;
   or
L234A, L235A and G237A.

In the present invention, letters before the position number represent amino acids before mutation, and letters after the position number represent amino acids after mutation, unless otherwise specified.

In one or more embodiments of the present invention, for the bispecific antibody, according to the EU numbering system, the heavy chain constant region of the immunoglobulin has or further has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A and K320A.

In one or more embodiments of the present invention, for the bispecific antibody, the heavy chain variable region of the immunoglobulin has an amino acid sequence set forth in SEQ ID NO: 31, and the light chain variable region of the immunoglobulin has an amino acid sequence set forth in SEQ ID NO: 33; the heavy chain variable region of the single chain antibody has an amino acid sequence set forth in SEQ ID NO: 41, and the light chain variable region of the single chain antibody has an amino acid sequence set forth in SEQ ID NO: 43.

In one or more embodiments of the present invention, for the bispecific antibody, the immunoglobulin comprises a heavy chain having an amino acid sequence set forth in SEQ ID NO: 27, and a light chain having an amino acid sequence set forth in SEQ ID NO: 29.

In some embodiments of the present invention, in the bispecific antibody, the single chain antibody is linked to the C terminus of the heavy chain of the immunoglobulin. Since an immunoglobulin has two heavy chains, two single chain antibody molecules are linked to one immunoglobulin molecule. Preferably, the two single chain antibody molecules are identical.

In some embodiments of the present invention, in the bispecific antibody, two single chain antibodies are present, and one terminus of each single chain antibody is linked to the C terminus or the N terminus of one of the two heavy chains of the immunoglobulin.

In some embodiments of the present invention, a disulfide bond is present between the V_{H} and the V_{L} of the single chain antibody. Methods for introducing disulfide bonds between the V_{H} and V_{L} of an antibody are well known in the art, see, for example, US5,747,654; Rajagopal, et al., Prot. Engin., 10(1997)1453-1459; Reiter et al., Nat. Biotechnol., 14(1996)1239-1245; Reiter, et al., Protein Engineering, 8(1995)1323-1331; Webber, et al., Molecular Immunology, 32(1995)249-258; Reiter, et al., Immunity, 2(1995)281-287; Reiter, et al., JBC, 269(1994)18327-18331; Reiter, et al., Inter. J. of Cancer, 58(1994)142-149; and Reiter, et al., Cancer Res., 54(1994)2714-2718, which are incorporated herein by reference.

In one or more embodiments of the present invention, in the bispecific antibody, the first protein functional region is linked to the second protein functional region either directly or via a linker fragment; and/or the heavy chain variable region of the single chain antibody is linked to the light chain variable region of the single chain antibody either directly or via a linker fragment.

In one or more embodiments of the present invention, in the bispecific antibody, the linker fragment is (GGGGS)n, wherein n is a positive integer; preferably, n is 1, 2, 3, 4, 5, or 6.

In one or more embodiments of the present invention, in the bispecific antibody, the numbers of the first protein functional region and the second protein functional region are each independently 1, 2, or more.

In one or more embodiments of the present invention, in the bispecific antibody, the single chain antibody is linked to the C terminus of the heavy chain of the immunoglobulin.

In one or more embodiments of the present invention, the first protein functional region is linked to the second protein functional region via a first linker fragment; and the heavy chain variable region of the single chain antibody is linked to the light chain variable region of the single chain antibody via a second linker fragment; the first linker fragment and the second linker fragment are the same or different;
preferably, the first linker fragment and the second linker fragment each have an amino acid sequence independently selected from SEQ ID NO: 52 and SEQ ID NO: 53;
preferably, the first linker fragment and second linker fragment both have an amino acid sequence set forth in SEQ ID NO: 53.

In one or more embodiments of the present invention, the bispecific antibody is a monoclonal antibody.

In one or more embodiments of the present invention, the bispecific antibody is a humanized antibody.

Another aspect of the present invention relates to a unit formulation, preferably used for treating a tumor, and comprising 1-10000 mg (preferably 10-1000 mg, preferably 50-500 mg, 100-400 mg, 150-300 mg, 150-250 mg, or 200 mg) of the anti-TIGIT antibody according to any aspect of the present invention, 1-10000 mg (preferably 1-1000 mg, preferably 50-500 mg, 100-400 mg, 150-300 mg, 150-250 mg, 200 mg, or 100 mg) of the anti-PD-1/anti-VEGFA bispecific antibody according to any aspect of the present invention, and optionally one or more of the therapeutic for treating tumors (such as chemotherapeutic, e.g., a platinum-based drug and/or a fluorouracil antineoplastic) according to the present invention; wherein the anti-TIGIT antibody, the anti-PD-1/anti-VEGFA bispecific antibody, and the therapeutic for treating tumors are packaged separately.

The present invention relates to a method for preventing or treating a cancer or a tumor, comprising administering to a subject in need one or more doses of the unit formulation according to the present invention, wherein preferably the anti-PD-1/anti-VEGFA bispecific antibody, the anti-TIGIT antibody, and the therapeutic for treating tumors in the unit formulation are each administered separately.

Another aspect of the present invention relates to a single dose unit, preferably used for treating a tumor, and comprising 0.1-10000 mg (preferably 1-1000 mg, preferably 50-500 mg, 100-400 mg, 150-300 mg, 150-250 mg, 200 mg or 100 mg) of the anti-TIGIT antibody according to any aspect of the present invention and 0.1-10000 mg (preferably 1-1000 mg, preferably 50-500 mg, 100-400 mg, 150-300 mg, 150-250 mg, 200 mg or 100 mg) of the anti-PD-1/anti-VEGFA bispecific antibody according to any aspect of the present invention.

In one or more embodiments of the present invention, the anti-TIGIT antibody, the anti-PD-1/anti-VEGFA bispecific antibody, and/or the therapeutic for treating tumors are in a form suitable for intravenous injection or intravenous drip infusion, preferably in a liquid form.

In one or more embodiments of the present invention, the administration of the effective amount of the anti-TIGIT antibody according to any aspect of the present invention and/or the anti-PD-1/anti-VEGFA bispecific antibody according to any aspect of the present invention to the subject is before or after a surgical treatment and/or before or after a radiation therapy.

In one or more embodiments of the present invention, the unit dose of the anti-TIGIT antibody according to any aspect of the present invention and/or the anti-PD-1/anti-VEGFA bispecific antibody according to any aspect of the present invention is 0.1-100 mg, preferably 1-10 mg, per kg body weight; alternatively, the unit dose of the anti-TIGIT antibody according to any aspect of the present invention and/or the anti-PD-1/anti-VEGFA bispecific antibody according to any aspect of the present invention is 10-1000 mg, preferably 50-500 mg, 100-400 mg, 150-300 mg, 150-250 mg, or 200 mg, in each subject,
preferably, the dose is administered from twice daily to about once every other day, or once every 3 days, 4 days, 5 days, 6 days, 10 days, 1 week, 2 weeks, 3 weeks, 4 weeks, 5 weeks, or 6 weeks;
preferably, the route of administration is intravenous drip infusion or intravenous injection.

The variable regions of the light chain and the heavy chain determine the antigen binding; the variable region of each chain comprises three hypervariable regions called complementarity determining regions (CDRs; CDRs of the heavy chain (H) include HCDR1, HCDR2, and HCDR3, and CDRs of the light chain (L) include LCDR1, LCDR2, and LCDR3, which are named by Kabat et al., see Bethesda M.d., Sequences of Proteins of Immunological Interest, Fifth Edition, NIH Publication 1991; 1-3:91-3242). Given the known sequences of the heavy and light chain variable regions of an antibody, there are several methods for determining the CDRs of the antibody, including the Kabat, IMGT, Chothia, and AbM numbering systems. However, the application of all the definitions of CDRs for an antibody or its variant shall fall within the scope of the terms defined and used herein. If an amino acid sequence of the variable region of the antibody is known, those skilled in the art can generally determine a particular CDR, without relying on any experimental data beyond the sequence itself.

Preferably, CDRs may also be defined by the IMGT numbering system, see Ehrenmann, Francois, Quentin Kaas, and Marie-Paule Lefranc., IMGT/3Dstructure-DB and IMGT/DomainGapAlign: a database and a tool for immunoglobulins or antibodies, T cell receptors, MHC, IgSF, and MhcSF. Nucleic acids research, 2009; 38(suppl_1): D301-D307.

The amino acid sequences of the CDRs of the monoclonal antibody sequences are analyzed according to the IMGT definition by technical means well known to those skilled in the art, for example by using the VBASE2 database.

The antibodies 26B12, 26B12H1L1, 26B12H4L1, 26B12H2L2, 26B12H3L2, 26B12H2L3, 26B12H3L3, 26B12H1L4, and 26B12H4L4 involved in the present invention have the same CDRs:
The 3 CDRs of the heavy chain variable region have the following amino acid sequences:
HCDR1: GHSFTSDYA (SEQ ID NO: 3)
HCDR2: ISYSDST (SEQ ID NO: 4)
HCDR3: ARLDYGNYGGAMDY (SEQ ID NO: 5);
the 3 CDRs of the light chain variable region have the following amino acid sequences:
   LCDR1: QHVSTA (SEQ ID NO: 8)
   LCDR2: SAS (SEQ ID NO: 9)
   LCDR3: QQHYITPWT (SEQ ID NO: 10).

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, when referring to the amino acid sequence of TIGIT (NCBI GenBank ID: NP_776160.2), it includes the full length of TIGIT protein, or an extracellular immunoglobulin variable region (IgV) domain or a fragment comprising an extracellular immunoglobulin variable region (IgV) domain; also included are fusion proteins of TIGIT, such as a fragment fused to an Fc protein fragment of a mouse or human IgG (mFc or hFc). However, those skilled in the art will appreciate that in the amino acid sequence of the TIGIT protein, mutations or variations (including but not limited to, substitutions, deletions, and/or additions) may naturally occur or can be artificially introduced without affecting biological functions thereof. Therefore, in the present invention, the term "TIGIT protein" or "TIGIT" shall include all such sequences, including the sequences set forth and natural or artificial variants thereof. Moreover, when describing the sequence fragment of the TIGIT protein, it includes not only the sequence fragment but also a corresponding sequence fragment in natural or artificial variants thereof.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified as κ and λ light chains. Heavy chains are classified as µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of classical complement system. The V_{H} and V_{L} regions can be further subdivided into hypervariable regions (called complementarity determining regions, or CDRs) and conservative regions called framework regions (FRs) that are distributed between the CDRs. Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (V_{H} and V_{L}) of each heavy chain/light chain pair form antigen-binding sites, respectively. The assignment of amino acids to the regions or domains is based on Kabat Sequences of Proteins of Immunological Interest (National Institutes of Health, Bethesda, M.d. (1987 and 1991)), or Chothia & Lesk, J. Mol. Biol., 1987; 196:901-917; Chothia et al., Nature, 1989; 342:878-883 or the definition of IMGT numbering system, see Ehrenmann, Francois, Quentin Kaas, and Marie-Paule Lefranc., "IMGT/3Dstructure-DB and IMGT/DomainGapAlign: a database and a tool for immunoglobulins or antibodies, T cell receptors, MHC, IgSF and MhcSF.", Nucleic acids research, 2009; 38(suppl_1): D301-D307. The term "antibody" is not limited by any specific method for producing the antibody. For example, the antibody includes, in particular, a recombinant antibody, a monoclonal antibody and a polyclonal antibody. Antibodies can be different isotypes of antibodies, such as antibodies IgG (e.g., subtype IgG1, IgG2, IgG3 or IgG4), IgA1, IgA2, IgD, IgE or IgM.

As used herein, the term "antigen-binding fragment" of an antibody, also known as the "antigen-binding portion", refers to a polypeptide comprising the fragment of a full-length antibody, which maintains the ability to specifically bind to the same antigen to which the full-length antibody binds, and/or competes with the full-length antibody for the specific binding to the antigen. See generally, Fundamental Immunology, Ch. 7 (Paul, W., ed., 2nd edition, Raven Press, N.Y. (1989), which is incorporated by reference herein in its entirety for all purposes. An antigen-binding fragment of an antibody can be produced by recombinant DNA technique or by enzymatic or chemical cleavage of an intact antibody. In some cases, the antigen-binding fragment includes Fab, Fab', F(ab')₂, Fd, Fv, dAb, and complementarity determining region (CDR) fragments, single chain antibody fragments (e.g., scFv), chimeric antibodies, diabodies, and polypeptides comprising at least a portion of the antibody sufficient to impart specific antigen binding ability.

As used herein, the term "Fd fragment" refers to an antibody fragment consisting of V_{H} and C_{H}1 domains; the term "Fv fragment" refers to an antibody fragment consisting of the V_{L} and V_{H} domains of a single arm of an antibody; the term "dAb fragment" refers to an antibody fragment consisting of a V_{H} domain (Ward et al., Nature, 341:544-546 (1989)); the term "Fab fragment" refers to an antibody fragment consisting of V_{L}, V_{H}, C_{L}, and C_{H}1 domains; and the term "F(ab')₂ fragment" refers to an antibody fragment comprising two Fab fragments linked by a disulfide bridge on a hinge region.

In some cases, the antigen-binding fragment of the antibody is a single chain antibody (e.g., scFv) in which the V_{L} and V_{H} domains are paired to form a monovalent molecule via a linker that enables them to produce a single polypeptide chain (see, e.g., Bird et al., Science, 242:423-426 (1988) and Huston et al., Proc. Natl. Acad. Sci. USA, 85:5879-5883 (1988)). Such scFv molecules may have a general structure: NH₂-V_{L}-linker-V_{H}-COOH or NH₂-V_{H}-linker-V_{L}-COOH. An appropriate linker in the prior art consists of GGGGS amino acid sequence repeats or a variant thereof. For example, a linker having the amino acid sequence (GGGGS)₄ may be used, but variants thereof may also be used (Holliger et al., (1993), Proc. Natl. Acad. Sci. USA, 90: 6444-6448). Other linkers that may be used in the present invention are described by Alfthan et al., (1995), Protein Eng., 8:725-731; Choi et al., (2001), Eur. J. Immunol., 31: 94-106; Hu et al., (1996), Cancer Res., 56:3055-3061; Kipriyanov et al., (1999), J. Mol. Biol., 293:41-56; and Roovers et al., (2001), Cancer Immunol*.*

In some cases, the antigen-binding fragment of the antibody is a diabody, that is, a bivalent antibody, in which the V_{H} and V_{L} domains are expressed on a single polypeptide chain. However, the linker used is too short to allow the pairing of the two domains on one chain, thereby the domains are forced to pair with the complementary domains on another chain and two antigen-binding sites are generated (see, e.g., Holliger P. et al., Proc. Natl. Acad. Sci. USA, 90:6444-6448 (1993), and Poljak R. J. et al., Structure, 2:1121-1123 (1994)).

In other cases, the antigen-binding fragment of the antibody is a "bispecific antibody", which refers to a conjugate formed from a first antibody (fragment) and a second antibody (fragment) or antibody analog via a linker; the methods of conjugation include, but are not limited to, chemical reaction, gene fusion, and enzyme catalysis. The antigen-binding fragment of the antibody may be a "multispecific antibody" including, for example, a trispecific antibody and a tetraspecific antibody, the former being an antibody with three different kinds of antigen-binding specificity, and the latter being an antibody with four different kinds of antigen-binding specificity. For example, a designed ankyrin repeat protein (DARPin) is linked to an IgG antibody, an scFv-Fc antibody fragment or combinations thereof, such as those described in CN104341529A. An anti-IL-17a fynomer binds to an anti-IL-6R antibody, such as those described in WO2015141862A1.

Antigen-binding fragments of antibodies (e.g., the antibody fragments described above) can be obtained from a given antibody (e.g., monoclonal antibody 26B12H1L1, 26B12H4L1, 26B12H2L2, 26B12H3L2, 26B12H2L3, 26B12H3L3, 26B12H1L4, or 26B12H4L4 provided in the present invention) using conventional techniques known to those skilled in the art (e.g., recombinant DNA technology or enzymatic or chemical cleavage), and the antigen-binding fragments of antibodies are screened for specificity in the same manner as for intact antibodies.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C., Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or antibody fragment obtained when all or a part of the CDRs of a human immunoglobulin (receptor antibody) are replaced by the CDRs of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321:522-525; Reichmann et al., Nature, 1988; 332:323-329; Presta, Curr. Op. Struct. Biol., 1992; 2:593-596; and Clark M., Antibody humanization: a case of the 'Emperor's new clothes' [J]. Immunol. Today, 2000; 21(8): 397-402.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages, such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, COS cells, NSO cells, HeLa cells, GS cells, BHK cells, HEK 293 cells, or human cells. As used herein, the term "specific binding" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) less than about 10⁻⁵ M, such as less than about 10⁻⁶ M, 10⁻⁷ M, 10⁻⁸ M, 10⁻⁹ M or 10⁻¹⁰ M or less.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, the antibodies bind to antigens (e.g., TIGIT protein) with a dissociation equilibrium constant (K_{D}) of less than about 10⁻⁵ M, e.g., less than about 10⁻⁶ M, 10⁻⁷ M, 10⁻⁸ M, 10⁻⁹ M or 10⁻¹⁰ M or less. K_{D} can be determined using methods known to those skilled in the art, e.g., using a Fortebio molecular interaction instrument.

As used herein, the terms "monoclonal antibody" and "mAb" have the same meaning and can be used interchangeably; the terms "polyclonal antibody" and "pAb" have the same meaning and can be used interchangeably; the terms "polypeptide" and "protein" have the same meaning and can be used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania: Mack Publishing Company, 1995), including but not limited to: pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop diseases and complications thereof in patients suffering from the disease.

As used herein, the terms "hybridoma" and "hybridoma cell line" can be used interchangeably, and when referring to the terms "hybridoma" and "hybridoma cell line", they further include subclones and progeny cells of the hybridoma.

The term "single dose unit" means a single pharmaceutical dosage form, such as an injection, e.g. placed in an ampoule, comprising the anti-TIGIT antibody and the anti-PD-1/anti-VEGFA bispecific antibody according to the present invention to be administered to a subject at time points of a regimen, preferably per kg body weight of the subject. In a specific embodiment of the present invention, the regimen comprises, for example, administration of a single dose unit according to an administration cycle of from twice daily to about once every other day, or once every 3 days, 4 days, 5 days, 6 days, 10 days, 1 week, 2 weeks, 3 weeks, 4 weeks, 5 weeks or 6 weeks.

In the present invention, the terms "first" (e.g., first protein functional region or first linker fragment) and "second" (e.g., second protein functional region or second linker fragment) are used for distinguishing or clarity in expression and do not carry typical sequential meanings, unless otherwise specified.

A "therapeutically effective amount" or "therapeutically effective dose" of a drug or therapeutic is any amount of a drug that, when used alone or in combination with another therapeutic, protects a subject from the onset of a disease or promotes disease regression as evidenced by the reduction in the severity of disease symptoms, increase in the frequency and duration of disease symptom-free periods, or the prevention of damage or disability caused by the affliction of the disease. The ability of a therapeutic to promote disease regression can be evaluated using a variety of methods known to those skilled in the art, such as in a human subject in clinical trials, in an animal model system that predicts the efficacy in humans, or by determining the activity of the drug in an *in vitro* assay.

The "prophylactically effective amount" of a drug refers to any amount of a drug that inhibits the occurrence or recurrence of cancer when administered, alone or in combination with an antineoplastic, to a subject at risk of developing a cancer (e.g., a subject having a premalignant condition) or a subject at risk of recurrence of a cancer. In some embodiments, the prophylactically effective amount completely prevents the occurrence or recurrence of a cancer. "Inhibiting" the occurrence or recurrence of cancer means reducing the possibility of the occurrence or recurrence of a cancer or completely preventing the occurrence or recurrence of a cancer.

### Beneficial Effects

The monoclonal antibodies of the present invention can specifically bind to TIGIT very well and have a strong affinity. They reduce the inhibitory effect of TIGIT on immune cells, promote the activity of T cells, reverse NK cell exhaustion, and enhance the killing effect of immune cells on tumors. The present invention can be used for preparing a medicament for inhibiting TIGIT or a medicament for treating or preventing diseases such as tumors in combination with an anti-PD-1/anti-VEGFA antibody, thus having good application prospects and market value.

### BRIEF DESCRIPTION OF THE DRAWINGS

FIG. 1: the results of assays for the activity of antibodies 26B12H1L1, 26B12H2L2, 26B12H2L3, and 26B12H3L2 binding to TIGIT-mFc.
FIG. 2: the results of assays for the activity of antibodies 26B12H3L3, 26B12H1L4, 26B12H4L1, and 26B12H4L4 binding to TIGIT-mFc.
FIG. 3: the results of assays for the activity of antibodies 26B12H1L1, 26B12H2L2, 26B12H2L3, and 26B12H3L2 competing with human CD155-hFc-Biotin for binding to TIGIT-mFc.
FIG. 4: the results of assays for the activity of antibodies 26B12H3L3, 26B12H1L4, 26B12H4L1, and 26B12H4L4 competing with human CD155-hFc-Biotin for binding to TIGIT-mFc.
FIG. 5: the results of assays for the affinity constant of 26B12H3L3 for TIGIT-mFc. The antibody concentrations for the curve pairs from top to bottom are 5 nM, 1.67 nM, 0.557 nM, 0.185 nM, and 0.06 nM, respectively.
FIG. 6: the results of assays for the affinity constant of 26B12H1L1 for TIGIT-mFc. The antibody concentrations for the curve pairs from top to bottom are 5 nM, 1.67 nM, 0.557 nM, 0.185 nM, and 0.06 nM, respectively.
FIG. 7: the results of assays for the affinity constant of 26B12H2L2 for TIGIT-mFc. The antibody concentrations for the curve pairs from top to bottom are 5 nM, 1.67 nM, 0.557 nM, 0.185 nM, and 0.06 nM, respectively.
FIG. 8: the results of assays for the affinity constant of 26B12H2L3 for TIGIT-mFc. The antibody concentrations for the curve pairs from top to bottom are 5 nM, 1.67 nM, 0.557 nM, 0.185 nM, and 0.06 nM, respectively.
FIG. 9: the results of assays for the affinity constant of 26B12H3L2 for TIGIT-mFc. The antibody concentrations for the curve pairs from top to bottom are 5 nM, 1.67 nM, 0.557 nM, 0.185 nM, and 0.06 nM, respectively.
FIG. 10: the results of assays for the affinity constant of 26B12H4L4 for TIGIT-mFc. The antibody concentrations for the curve pairs from top to bottom are 5 nM, 1.67 nM, 0.557 nM, 0.185 nM, and 0.06 nM, respectively.
FIG. 11: the results of assays for the affinity constant of 26B12H1L4 for TIGIT-mFc. The antibody concentrations for the curve pairs from top to bottom are 5 nM, 1.67 nM, 0.557 nM, 0.185 nM, and 0.06 nM, respectively.
FIG. 12: the results of assays for the affinity constant of 26B12H4L1 for TIGIT-mFc. The antibody concentrations for the curve pairs from top to bottom are 5 nM, 1.67 nM, 0.557 nM, 0.185 nM, and 0.06 nM, respectively.
FIG. 13: the results of assays for the affinity constant of RG6058 for TIGIT-mFc. The antibody concentrations for the curve pairs from top to bottom are 5 nM, 1.67 nM, 0.557 nM, 0.185 nM, and 0.06 nM, respectively.
FIG. 14: FACS assays for the activity of humanized antibodies 26B12H2L2 and RG6058 binding to 293T-TIGIT cell membrane surface antigen TIGIT.
FIG. 15: FACS assays for the activity of humanized antibodies 26B12H2L2 and RG6058 competing with CD155 for binding to 293T-TIGIT cell membrane surface TIGIT.
FIG. 16: FACS assays for the activity of humanized antibodies 26B12H2L2 and RG6058 competing with CD112 for binding to 293T-TIGIT cell membrane surface TIGIT.
FIG. 17: the effect on hTIGIT-BALB/c transgenic mouse CT26 tumor model.
FIG. 18: the body weight changes of hTIGIT-BALB/c transgenic mouse CT26 tumor model.
FIG. 19: the efficacy of 26B12H2L2 in combination with anti-PD-1/anti-VEGFA bispecific antibody VP101(hG1DM) on a BALB/c-hPD1/hTIGIT transgenic mouse CT26 tumor model.
FIG. 20: the body weight changes in a BALB/c-hPD1/hTIGIT transgenic mouse CT26 tumor model receiving 26B12H2L2 in combination with anti-PD-1/anti-VEGFA bispecific antibody VP101(hG1DM).
FIG. 21: anti-TIGIT antibody in combination with anti-PD-1/anti-VEGFA bifunctional antibody effectively neutralizing the inhibition of the signaling pathways mediated by the binding of the corresponding targets to the receptors.

### Deposited biological material:

Hybridoma cell line LT019 (TIGIT-26B12) was deposited at China Center for Type Culture Collection (CCTCC) on Oct. 23, 2020, under CCTCC NO. C2020208, the depository address being Wuhan University, Wuhan, China, postal code: 430072.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Guide to Molecular Cloning Experiments, authored by J. Sambrook et al., and translated by Huang Peitang et al., third edition, Science Press) or according to the product instruction. Reagents or instruments used are commercially available conventional products if the manufacturers thereof are not specified. For example, 293T can be purchased from ATCC.

In the following examples of the present invention, BALB/c mice used were purchased from Guangdong Medical Experimental Animal Center.

In the following examples of the present invention, the positive control antibody RG6058 has a sequence that can be found in the sequence 34 and the sequence 36 in the Chinese Patent Publication No. CN108290946.

In the following examples of the present invention, the anti-PD-1/anti-VEGFA bispecific antibody VP101(hG1DM) used in the combination was produced by Akeso Biopharma, Inc., the sequence of which can be found in Publication Patent No. CN112830972A, wherein the full-length amino acid sequence of the heavy chain of VP101(hG1DM) is set forth in SEQ ID NO: 27 and the full-length amino acid sequence of the light chain thereof is set forth in SEQ ID NO: 29. VP101(hG1DM) has a structure of IgG-scFv, with the IgG part being an anti-VEGFA antibody and the scFv part being an anti-PD-1 antibody, wherein
the anti-VEGFA antibody has an HCDR1 sequence set forth in SEQ ID NO: 35, an HCDR2 sequence set forth in SEQ ID NO: 36, an HCDR3 sequence set forth in SEQ ID NO: 37, and a VH sequence set forth in SEQ ID NO: 31, and the anti-VEGFA antibody has an LCDR1 sequence set forth in SEQ ID NO: 38, an LCDR2 sequence set forth in SEQ ID NO: 39, an LCDR3 sequence set forth in SEQ ID NO: 40, and a VL sequence set forth in SEQ ID NO: 33; and
the anti-PD1 antibody has an HCDR1 sequence set forth in SEQ ID NO: 45, an HCDR2 sequence set forth in SEQ ID NO: 46, an HCDR3 sequence set forth in SEQ ID NO: 47, and a VH sequence set forth in SEQ ID NO: 41, and the anti-PD1 antibody has an LCDR1 sequence set forth in SEQ ID NO: 48, an LCDR2 sequence set forth in SEQ ID NO: 49, an LCDR3 sequence set forth in SEQ ID NO: 50, and a VL sequence set forth in SEQ ID NO: 43.

The variable region sequence of the isotype control antibody in the examples of the present invention, human anti-hen egg lysozyme IgG (anti-HEL, or human IgG, abbreviated as hIgG), is described by Acierno et al., "Affinity maturation increases the stability and plasticity of the Fv domain of anti-protein antibodies" (Acierno et al., J Mol Biol., 2007; 374(1):130-46). The hIgGIDM used in the examples is an isotype control antibody with anti-HEL having a hGIDM constant region sequence (SEQ ID NO: 55), prepared in Akeso Biopharma, Inc.

In the following examples of the present invention, the cell line CHO-aAPC-PDL1-PVR used was constructed by Akeso Biopharma, Inc. The cell line CHO-aAPC-PDL1-PVR was prepared by viral infection of PD-L1 aAPC/CHO-K1 cells (purchased from Promega) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was pCDH-PVRFL-Puro (PVRFL, Genebank ID: NP_006496.4; vector pCDH-CMV-MCS-EF1-Puro, purchased from Youbio, Cat. No. VT1480).

In the following examples of the present invention, the cell line Jurkat-NFAT-PD1-TIGIT used was constructed by Akeso Biopharma, Inc. The cell line Jurkat-NFAT-PD1-TIGIT was prepared by viral infection of PD-1 effector cells (purchased from Promega) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11):8463-8471), wherein the lentivirus expression vector used was plenti6.3/V5-TIGITFL-BSD (TIGIT, Genebank ID: NP_776160.2; vector plenti6.3/V5 TOPO, purchased from Invitrogen, Cat. No. K531520).

### Example 1: Preparation of anti-TIGIT antibody 26B12

### 1. Preparation of hybridoma cell line LT019

The antigen used for preparing the anti-TIGIT antibody was human TIGIT-mFc (TIGIT was GenbankID: NP_776160.2; the mFc sequence is set forth in SEQ ID NO: 51). Spleen cells of immunized mice were fused with myeloma cells of the mice to prepare hybridoma cells. With human TIGIT-mFc taken as antigens, the hybridoma cells were screened by indirect ELISA to obtain hybridoma cells capable of secreting antibodies specifically binding to TIGIT. The hybridoma cells obtained by screening were subjected to limiting dilution to obtain a stable hybridoma cell line. The above hybridoma cell line was designated hybridoma cell line LT019, and the monoclonal antibody secreted by the cell line was designated 26B12.

Hybridoma cell line LT019 (or TIGIT-26B12) was deposited at China Center for Type Culture Collection (CCTCC) on Oct. 23, 2020, under CCTCC NO. C2020208, the depository address being Wuhan University, Wuhan, China, postal code: 430072.

### 2. Preparation of anti-TIGIT antibody 26B12

The LT019 cell line prepared above was cultured in a chemical defined medium (CD medium, containing 1% penicillin-streptomycin) at 37 °C/5% CO₂. After 7 days, the cell culture supernatant was collected, subjected to high-speed centrifugation and vacuum filtration through a microfiltration membrane, and purified by using a HiTrap protein A HP column to obtain antibody 26B12.

### Example 2: Sequence analysis of anti-TIGIT antibody 26B12

mRNA was extracted from the cell line LT019 cultured in Example 1 according to the method described in the manual of RNAprep pure Cell/Bacteria Kit (Tiangen, Cat. No. DP430).

cDNA was synthesized according to the manual of Invitrogen SuperScript^{®} III First-Strand Synthesis System for RT-PCR and amplified by PCR.

The PCR-amplified products were directly subjected to TA cloning according to the manual of the pEASY-T1 Cloning Kit (Transgen CT101).

The TA-cloned products were directly sequenced, and the sequencing results are as follows:
The nucleotide sequence of the heavy chain variable region is set forth in SEQ ID NO: 2 with a length of 363 bp.

The encoded amino acid sequence is set forth in SEQ ID NO: 1 with a length of 121 amino acids,
wherein the sequences of heavy chain HCDR1, HCDR2, and HCDR3 are set forth in SEQ ID NOs: 3, 4, and 5, respectively.

The nucleotide sequence of the light chain variable region is set forth in SEQ ID NO: 7 with a length of 321 bp.

The coded amino acid sequence is set forth in SEQ ID NO: 6 with a length of 107 amino acids,
wherein the sequences of light chain LCDR1, LCDR2, and LCDR3 are set forth in SEQ ID NOs: 8, 9, and 10, respectively.

### Example 3: Design and preparation of light and heavy chains of humanized anti-human TIGIT antibodies

### 1. Design of light and heavy chains of humanized anti-human TIGIT antibodies 26B12H1L1, 26B12H4L1, 26B12H2L2, 26B12H3L2, 26B12H2L3, 26B12H3L3, 26B12H1L4, and 26B12H4L4

Based on the three-dimensional crystal structure of human TIGIT protein and the sequence of antibody 26B12 obtained in Example 2, the antibody model was simulated by computer, and mutations were designed according to the model to obtain the variable region sequences of antibodies 26B12H1L1, 26B12H4L1, 26B12H2L2, 26B12H3L2, 26B12H2L3, 26B12H3L3, 26B12H1L4, and 26B12H4L4 (antibody constant region sequences from the NCBI database: the heavy chain constant region is Ig gamma-1 chain C region, ACCESSION: P01857; the light chain constant region is Ig kappa chain C region, ACCESSION: P01834).

The designed variable region sequences are shown in Table A below.

| No. | Name | Heavy chain variable region amino acid sequence SEQ ID NO: | Heavy chain variable region nucleotide sequence SEQ ID NO: | Light chain variable region amino acid sequence SEQ ID NO: | Light chain variable region nucleotide sequence SEQ ID NO: |
|---|---|---|---|---|---|
| 1 | 26B12H1L1 | 11 | 12 | 19 | 20 |
| 2 | 26B12H4L1 | 17 | 18 | 19 | 20 |
| 3 | 26B12H2L2 | 13 | 14 | 21 | 22 |
| 4 | 26B12H2L3 | 13 | 14 | 23 | 24 |
| 5 | 26B12H3L2 | 15 | 16 | 21 | 22 |
| 6 | 26B12H3L3 | 15 | 16 | 23 | 24 |
| 7 | 26B12H1L4 | 11 | 12 | 25 | 26 |
| 8 | 26B12H4L4 | 17 | 18 | 25 | 26 |

### (7) Heavy and light chain variable region sequences of humanized monoclonal antibody 26B12H1L4

For the above 8 antibodies 26B12H1L1, 26B12H4L1, 26B12H2L2, 26B12H3L2, 26B12H2L3, 26B12H3L3, 26B12H1L4, and 26B12H4L4, the length of the nucleotide sequences of the heavy chain variable regions was 363 bp, and the length of the encoded amino acid sequences was 121 aa; the length of the nucleotide sequences of the light chain variable regions was 321 bp, and the length of the encoded amino acid sequences was 107 aa.

Moreover, the above 8 antibodies had the same HCDR1-HCDR3 and LCDR1-LCDR3:
the sequences of HCDR1, HCDR2, and HCDR3 are set forth in SEQ ID NOs: 3, 4, and 5, respectively;
the sequences of LCDR1, LCDR2, and LCDR3 are set forth in SEQ ID NOs: 8, 9, and 10, respectively.

### 2. Preparation of humanized antibodies 26B12H1L1, 26B12H4L1, 26B12H2L2, 26B12H3L2, 26B12H2L3, 26B12H3L3, 26B12H1L4, and 26B12H4L4

The heavy chain constant regions were all Ig gamma-1 chain C region, ACCESSION: P01857; the light chain constant regions were all Ig kappa chain C region, ACCESSION: P01834.

Heavy chain cDNA and light chain cDNA of 26B12H1L1, heavy chain cDNA and light chain cDNA of 26B12H4L1, heavy chain cDNA and light chain cDNA of 26B12H2L2, heavy chain cDNA and light chain cDNA of 26B12H3L2, heavy chain cDNA and light chain cDNA of 26B12H2L3, heavy chain cDNA and light chain cDNA of 26B12H3L3, heavy chain cDNA and light chain cDNA of 26B12H1L4, heavy chain cDNA and light chain cDNA of 26B12H2L4, and heavy chain cDNA and light chain cDNA of 26B12H4L4 were separately cloned into pUC57simple (provided by GenScript) vectors to obtain pUC57simple-26B12H1, pUC57simple-26B12L1; pUC57simple-26B12H4, pUC57simple-26B12L1; pUC57simple-26B12H2, pUC57simple-26B12L2; pUC57simple-26B12H3, pUC57simple-26B12L2; pUC57simple-26B12H2, pUC57simple-26B12L3; pUC57simple-26B12H3, pUC57simple-26B12L3; pUC57simple-26B12H1, pUC57simple-26B12L4; pUC57simple-26B12H2, pUC57simple-26B12L4; and pUC57simple-26B12H4, pUC57simple-26B12L4, respectively. With reference to the standard techniques described in *Molecular Cloning: A Laboratory Manual* (Second Edition), the heavy and light chain full-length genes synthesized by EcoRI&HindIII digestion were subcloned into expression vector pcDNA3.1 by digestion with restriction enzyme EcoRI&HindIII to obtain expression plasmids pcDNA3.1-26B12H1, pcDNA3.1-26B12L1, pcDNA3.1-26B12H4, pcDNA3.1-126B12H2, pcDNA3.1-26B12L2, pcDNA3.1-26B12H3, pcDNA3.1-26B12L3 and pcDNA3.1-26B12L4, and the heavy/light chain genes of the recombinant expression plasmids were further sequenced. Then the designed gene combinations comprising corresponding light and heavy chain recombinant plasmids (pcDNA3 .1-26B 12H1/pcDNA3.1-26B12L1, pcDNA3.1-26B12H4/pcDNA3.1-26B12L1, pcDNA3.1-26B12H2/pcDNA3.1-26B12L2, pcDNA3.1-26B12H3/pcDNA3.1-26B12L2, pcDNA3.1-26B12H2/pcDNA3.1-26B12L3, pcDNA3.1-26B12H3/pcDNA3.1-26B12L3, pcDNA3.1-26B12Hl/pcDNA3.1-26B12L4 and pcDNA3.1-26B12H4/pcDNA3.1-26B12L4) were separately co-transfected into 293F cells, and the cultures were collected and purified. After the sequences were verified, endotoxin-free expression plasmids were prepared, and were transiently transfected into HEK293 cells for antibody expression. After 7 days, the cell cultures were collected and subjected to affinity purification on a Protein A column to obtain humanized antibodies.

### Example 4: ELISA assays for activity of antibodies binding to antigen TIGIT-mFc

Procedures: Goat anti-mouse IgG Fc (purchased from Jackson, lot no. 132560) was added at 2 µg/mL to a microplate and incubated at 4 °C for 16 h. After incubation, the microplate coated with goat anti-mouse IgG Fc was washed once with PBST, and then blocked with a PBST solution containing 1% BSA as a microplate blocking solution for 2 h. After blocking, the microplate was washed 3 times with PBST. Then, 1 µg/mL antigen human TIGIT-mFc was added, and the plate was incubated at 37 °C for 30 min and then washed 3 times with PBST. The antibodies serially diluted with the PBST solution were added to the wells of the microplate. The antibody dilution gradients are shown in Table 1 and Table 2. The microplate containing the test antibodies was incubated at 37 °C for 30 minutes, and then washed 3 times with PBST. After washing, HRP-labeled goat anti-human IgG Fc (purchased from Jackson, lot no. 128332) secondary antibody working solution diluted in a ratio of 1:5000 was added, and the microplate was incubated at 37 °C for 30 min. After incubation, the plate was washed 4 times with PBST, TMB (Neogen, 308177) was added for chromogenesis in the dark for 4 min, and then a stop solution was added to terminate the chromogenic reaction. Then the microplate was put into a microplate reader immediately, and the OD value of each well in the microplate was read at 450 nm. The data were analyzed by SoftMax Pro 6.2.1.

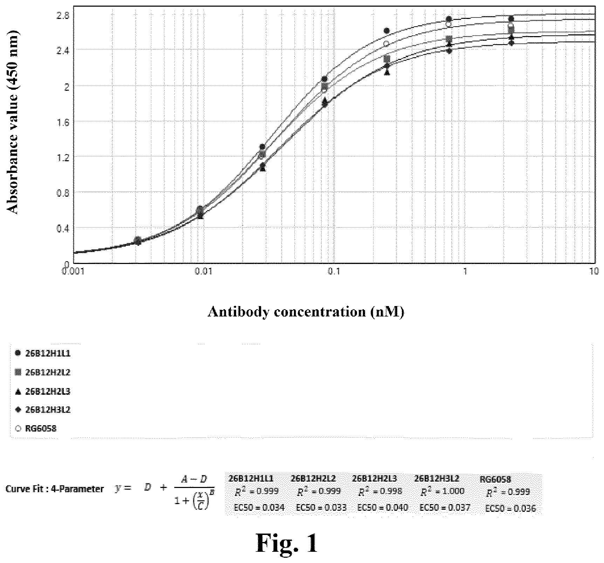

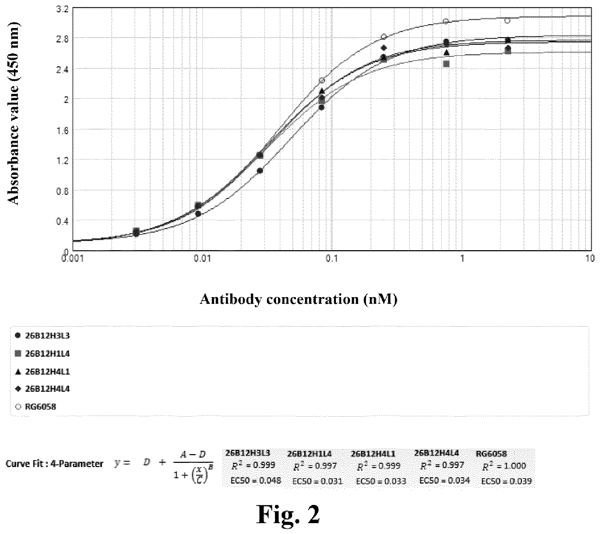

The results of the binding of the antibodies to the antigen TIGIT-mFc are shown in FIGs. 1 and 2. The OD values for all the dosages are shown in Table 1 and Table 2. An absorbance vs. antibody concentration curve was fitted, and the EC₅₀ of the antibodies binding to the antigen was calculated. The results are shown in Table 1 and Table 2, and FIG. 1 and FIG. 2.

**Table 1: The results of assays for the activity of 26B12H1L1, 26B12H2L2, 26B12H2L3, 26B12H3L2, and RG6058 binding to TIGIT-mFc**

| Coating: goat anti-mouse IgG Fc (2 µg/mL) | | | | | | | | | | |
|---|---|---|---|---|---|---|---|---|---|---|
| Antibody dilution (µg/mL) | TIGIT-mFc (1µg/mL) | | | | | | | | | |
| | 26B12H1L1 | | 26B12H2L2 | | 26B12H2L3 | | 26B12H3L2 | | RG6058 | |
| 0.3330 | 2.789 | 2.701 | 2.623 | 2.609 | 2.560 | 2.542 | 2.513 | 2.430 | 2.644 | 2.689 |
| 0.1110 | 2.722 | 2.771 | 2.460 | 2.572 | 2.413 | 2.528 | 2.368 | 2.394 | 2.516 | 2.863 |
| 0.0370 | 2.539 | 2.676 | 2.322 | 2.253 | 2.162 | 2.137 | 2.142 | 2.292 | 2.332 | 2.605 |
| 0.0123 | 2.001 | 2.126 | 2.001 | 1.965 | 1.853 | 1.811 | 1.706 | 1.861 | 1.855 | 2.028 |
| 0.0041 | 1.262 | 1.349 | 1.211 | 1.221 | 1.038 | 1.103 | 1.106 | 1.092 | 1.142 | 1.263 |
| 0.0014 | 0.593 | 0.613 | 0.542 | 0.627 | 0.506 | 0.544 | 0.546 | 0.527 | 0.569 | 0.602 |
| 0.0005 | 0.264 | 0.265 | 0.249 | 0.258 | 0.237 | 0.243 | 0.224 | 0.229 | 0.238 | 0.254 |
| 0.0000 | 0.056 | 0.051 | 0.053 | 0.046 | 0.052 | 0.049 | 0.049 | 0.050 | 0.053 | 0.049 |

| Secondary antibody | HRP-labeled goat anti-human IgG Fc (1:5000) | | | | | | | | | |
|---|---|---|---|---|---|---|---|---|---|---|
| EC₅₀(nM) | 0.034 | | 0.033 | | 0.040 | | 0.037 | | 0.036 | |

**Table 2: The results of assays for the activity of 26B12H3L3, 26B12H1L4, 26B 12H4L1, 26B12H4L4, and RG6058 binding to TIGIT-mFc**

| Coating: goat anti-mouse IgG Fc (2 µg/mL) | | | | | | | | | | |
|---|---|---|---|---|---|---|---|---|---|---|
| Antibody dilution (µg/mL) | TIGIT-mFc (1µg/mL) | | | | | | | | | |
| | 26B12H3L3 | | 26B12H1L4 | | 26B12H4L1 | | 26B12H4L4 | | RG6058 | |
| 0.3330 | 2.736 | 2.788 | 2.639 | 2.604 | 2.709 | 2.829 | 2.728 | 2.608 | 2.963 | 3.089 |
| 0.1110 | 2.707 | 2.774 | 2.469 | 2.422 | 2.625 | 2.587 | 2.626 | 2.788 | 2.915 | 3.119 |
| 0.0370 | 2.546 | 2.538 | 2.568 | 2.451 | 2.392 | 2.699 | 2.679 | 2.660 | 2.830 | 2.797 |
| 0.0123 | 1.861 | 1.881 | 2.049 | 1.882 | 2.113 | 2.091 | 1.987 | 2.045 | 2.237 | 2.237 |
| 0.0041 | 1.074 | 1.012 | 1.232 | 1.266 | 1.252 | 1.279 | 1.265 | 1.239 | 1.254 | 1.258 |
| 0.0014 | 0.483 | 0.477 | 0.593 | 0.580 | 0.582 | 0.592 | 0.589 | 0.593 | 0.569 | 0.593 |
| 0.0005 | 0.217 | 0.211 | 0.246 | 0.263 | 0.256 | 0.261 | 0.253 | 0.253 | 0.244 | 0.248 |
| 0.0000 | 0.065 | 0.060 | 0.053 | 0.051 | 0.051 | 0.051 | 0.052 | 0.054 | 0.065 | 0.061 |

| Secondary antibody | HRP-labeled goat anti-human IgG Fc (1:5000) | | | | | | | | | |
|---|---|---|---|---|---|---|---|---|---|---|
| EC₅₀(nM) | 0.048 | | 0.031 | | 0.033 | | 0.034 | | 0.039 | |

The results show that the antibodies 26B12H1L1, 26B12H4L1, 26B12H2L2, 26B12H3L2, 26B12H2L3, 26B12H3L3, 26B12H1L4, and 26B12H4L4 are all capable of effectively binding to human TIGIT-mFc in a dose-dependent manner, and the binding activities are comparable to that of the positive drug RG6058 for the same target, indicating that the antibodies 26B12H1L1, 26B12H4L1, 26B12H2L2, 26B12H3L2, 26B12H2L3, 26B12H3L3, 26B12H1L4, and 26B12H4L4 have the function of effectively binding to TIGIT.

### Example 5: Competitive ELISA assays for activity of antibodies competing with CD155-hFc-biotin for binding to TIGIT-mFc

Procedures: a microplate was coated with TIGIT-mFc at 2 µg/mL, and incubated at 4 °C overnight. After incubation, the antigen-coated microplate was rinsed once with PBST, and then blocked with a PBST solution containing 1% BSA as a microplate blocking solution for 2 h. After blocking, the microplate was washed 3 times with PBST. The antibodies serially diluted with the PBST solution were added to the microplate. The antibody concentrations are shown in Table 3 and Table 4. After the microplate was incubated at room temperature for 10 min, equal volumes of 2 µg/mL CD155-hFc-Biotin (produced by Akeso Biopharma, Inc., lot no. 20170210, wherein CD155 was GenBank accession no. NP_006496.4, and the hFc sequence is set forth in SEQ ID NO: 54; final concentration: 1 µg/mL) were added, and mixed well with the antibodies. The microplate was incubated at 37 °C for 30 min, and then washed 3 times with PBST. After washing, SA-HRP working solution diluted in a ratio of 1:4000 was added, and the microplate was incubated at 37 °C for 30 min. After incubation, the plate was washed 4 times with PBST, TMB (Neogen, 308177) was added for chromogenesis in the dark for 5 min, and then a stop solution was added to terminate the chromogenic reaction. Then the microplate was put into a microplate reader immediately, and the OD value of each well in the microplate was read at 450 nm. The data were analyzed by SoftMax Pro 6.2.1.

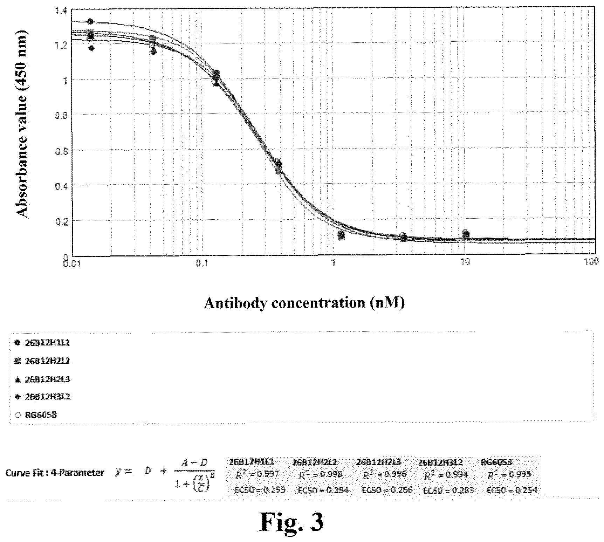

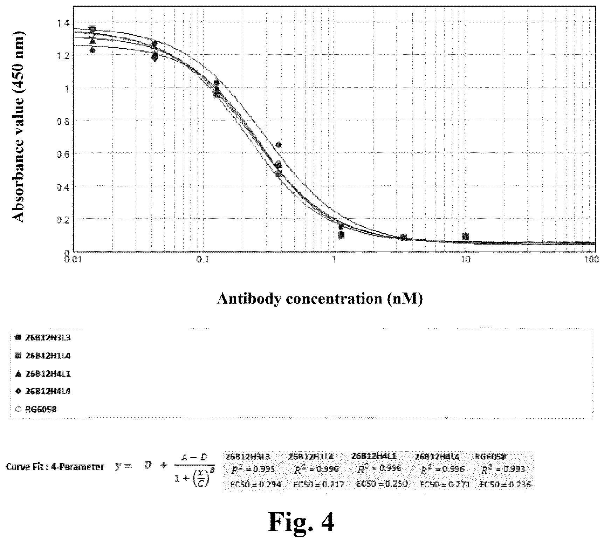

The results of the activity of the antibodies competing with CD155-hFc-Biotin for binding to TIGIT-mFc are shown in Table 3 and Table 4. An absorbance vs. antibody concentration curve was fitted, and the EC₅₀ of the antibodies competing with CD155-hFc-Biotin for binding to TIGIT-mFc was calculated. The results are shown in Table 3 and Table 4, and FIG. 3 and FIG. 4 below.

**Table 3: The results of assays for the activity of 26B12H1L1, 26B12H2L2, 26B12H2L3, 26B12H3L2, and RG6058 competing with human CD155-hFc-Biotin for binding to TIGIT-mFc**

| Antibody dilution | Antigen coating: TIGIT-mFc (2 µg/mL) | | | | | | | | | |
|---|---|---|---|---|---|---|---|---|---|---|
| | 26B12H1L1 | | 26B12H2L2 | | 26B12H2L3 | | 26B12H3L2 | | RG6058 | |
| 3 µg/mL | 0.091 | 0.097 | 0.101 | 0.110 | 0.106 | 0.117 | 0.107 | 0.113 | 0.119 | 0.120 |
| 1:3 | 0.088 | 0.085 | 0.082 | 0.092 | 0.098 | 0.100 | 0.101 | 0.102 | 0.104 | 0.112 |
| 1:9 | 0.104 | 0.099 | 0.091 | 0.097 | 0.107 | 0.110 | 0.121 | 0.120 | 0.114 | 0.121 |
| 1:27 | 0.533 | 0.491 | 0.410 | 0.538 | 0.510 | 0.537 | 0.492 | 0.549 | 0.528 | 0.532 |
| 1:81 | 1.026 | 1.035 | 0.996 | 1.025 | 0.961 | 0.990 | 0.948 | 1.059 | 0.951 | 1.011 |
| 1:243 | 1.210 | 1.251 | 1.222 | 1.221 | 1.142 | 1.195 | 1.089 | 1.217 | 1.168 | 1.209 |
| 1:729 | 1.287 | 1.360 | 1.274 | 1.242 | 1.201 | 1.287 | 1.120 | 1.236 | 1.209 | 1.251 |
| 0 | 1.347 | 1.387 | 1.315 | 1.296 | 1.279 | 1.307 | 1.263 | 1.340 | 1.295 | 1.354 |
| CD155-hFc-Biotin (1µg/mL) | | | | | | | | | | |
| SA-HRP (1:4000) | | | | | | | | | | |
| EC₅₀ (nM) | 0.255 | | 0.254 | | 0.266 | | 0.283 | | 0.254 | |

**Table 4: The results of assays for the activity of 26B12H3L3, 26B12H1L4, 26B12H4L1, 26B12H4L4, and RG6058 competing with human CD155-hFc-Biotin for binding to TIGIT-mFc**

| Antibody dilution | Antigen coating: TIGIT-mFc (2 µg/mL) | | | | | | | | | |
|---|---|---|---|---|---|---|---|---|---|---|
| | 26B12H3L3 | | 26B12H1L4 | | 26B12H4L1 | | 26B12H4L4 | | RG6058 | |
| 3 µg/mL | 0.087 | 0.085 | 0.091 | 0.084 | 0.102 | 0.086 | 0.091 | 0.085 | 0.091 | 0.093 |
| 1:3 | 0.083 | 0.082 | 0.077 | 0.086 | 0.086 | 0.086 | 0.084 | 0.082 | 0.089 | 0.084 |
| 1:9 | 0.139 | 0.146 | 0.094 | 0.096 | 0.106 | 0.102 | 0.103 | 0.103 | 0.101 | 0.100 |
| 1:27 | 0.646 | 0.650 | 0.449 | 0.495 | 0.495 | 0.561 | 0.516 | 0.532 | 0.530 | 0.539 |
| 1:81 | 1.031 | 1.027 | 0.938 | 0.967 | 0.931 | 1.030 | 0.974 | 0.999 | 0.946 | 1.037 |
| 1:243 | 1.239 | 1.294 | 1.171 | 1.224 | 1.180 | 1.232 | 1.131 | 1.218 | 1.152 | 1.223 |
| 1:729 | 1.318 | 1.378 | 1.336 | 1.382 | 1.278 | 1.292 | 1.123 | 1.335 | 1.302 | 1.346 |
| 0 | 1.410 | 1.393 | 1.341 | 1.361 | 1.357 | 1.360 | 1.258 | 1.364 | 1.380 | 1.427 |
| CD155-hFc-Biotin (1µg/mL) | | | | | | | | | | |
| SA-HRP (1:4000) | | | | | | | | | | |
| EC₅₀ (nM) | 0.294 | | 0.217 | | 0.250 | | 0.271 | | 0.236 | |

The results show that in the same experimental conditions, 26B12H1L1, 26B12H4L1, 26B12H2L2, 26B12H3L2, 26B12H2L3, 26B12H3L3, 26B12H1L4, and 26B12H4L4 can compete with CD155-hFc-Biotin for binding to the antigen TIGIT-mFc, and the activities are comparable to that of the positive drug RG6058 for the same target, indicating that 26B12H1L1, 26B12H4L1, 26B12H2L2, 26B12H3L2, 26B12H2L3, 26B12H3L3, 26B12H1L4, and 26B12H4L4 have an effective function of competing with CD155-hFc-Biotin for binding to TIGIT-mFc.

### Example 6: Assays for kinetics of humanized antibodies 26B12H3L3, 26B12H1L1, 26B12H2L2, 26B12H2L3, 26B12H3L2, 26B12H4L4, 26B12H1L4, 26B12H4L1 and RG6058 binding to antigen TIGIT-mFc using Fortebio molecular interaction instrument

The sample dilution buffer was PBS (0.02% Tween-20, 0.1% BSA, pH 7.4). TIGIT-mFc was immobilized on an AMC sensor at a concentration of 3 µg/mL for 50 s, and the sensor was equilibrated in the buffer for 60 s. The TIGIT-mFc immobilized on the sensor was allowed to bind to the antibody at concentrations of 0.06-5 nM (three-fold dilutions) for 120 s, and the protein was dissociated in the buffer for 300 s. The sensor was regenerated in 10 mM glycine solution (pH = 1.7). The detection temperature was 37 °C, the detection frequency was 0.3 Hz, and the sample plate shaking rate was 1000 rpm. The data were analyzed by 1: 1 model fitting to obtain affinity constants.

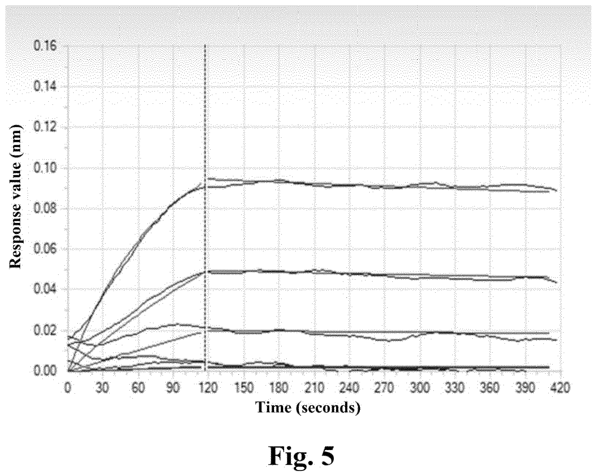

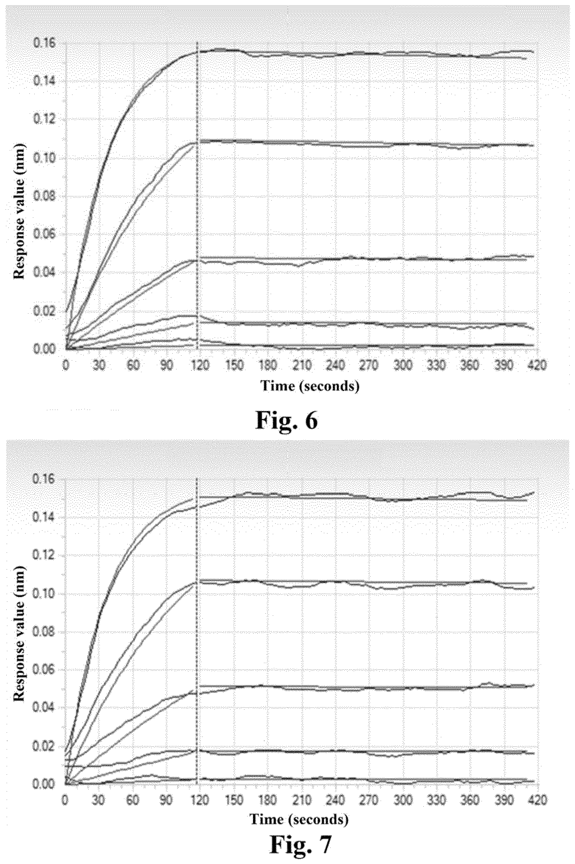

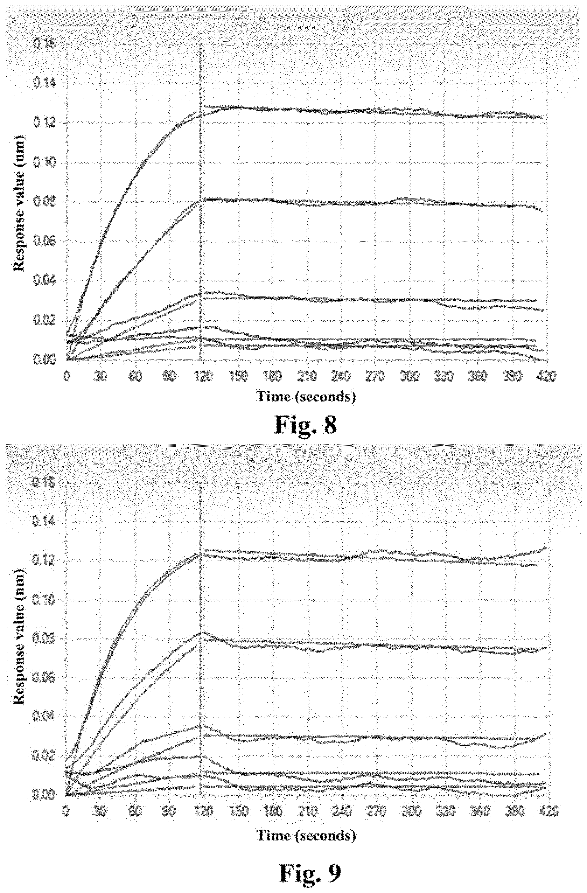

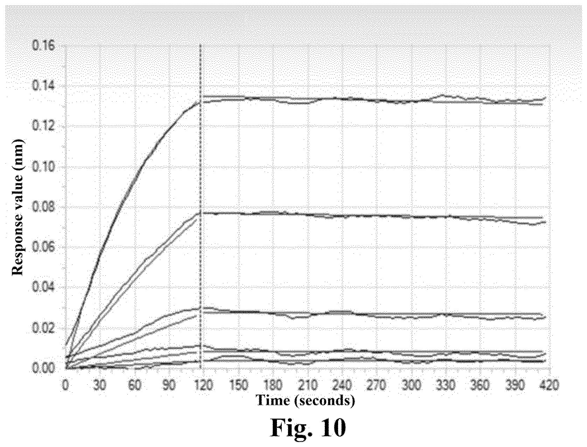

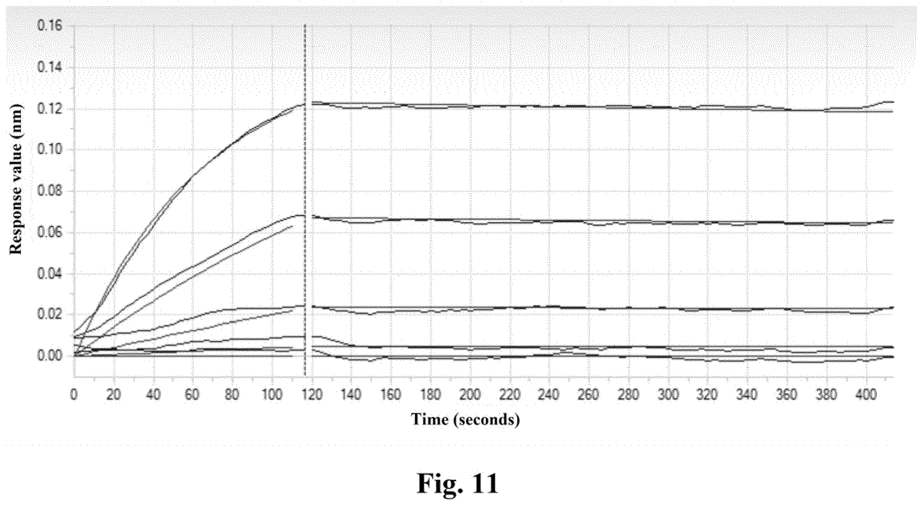

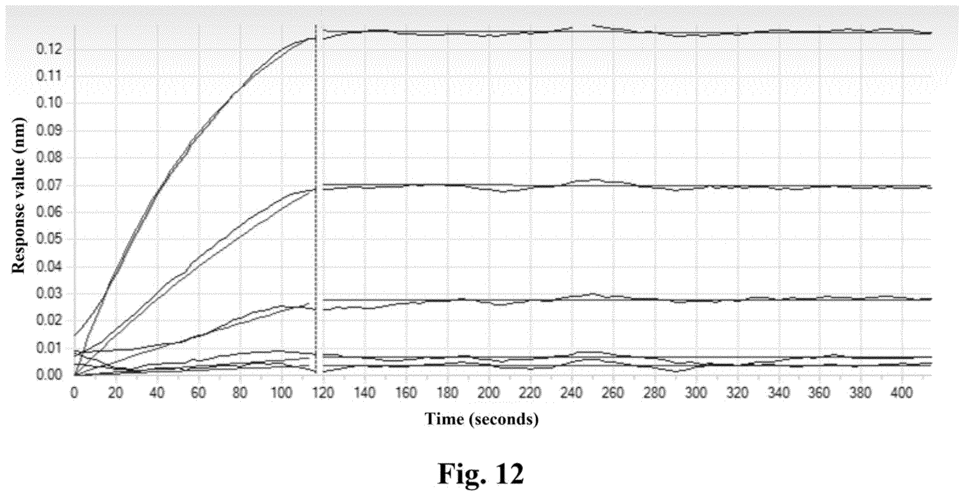

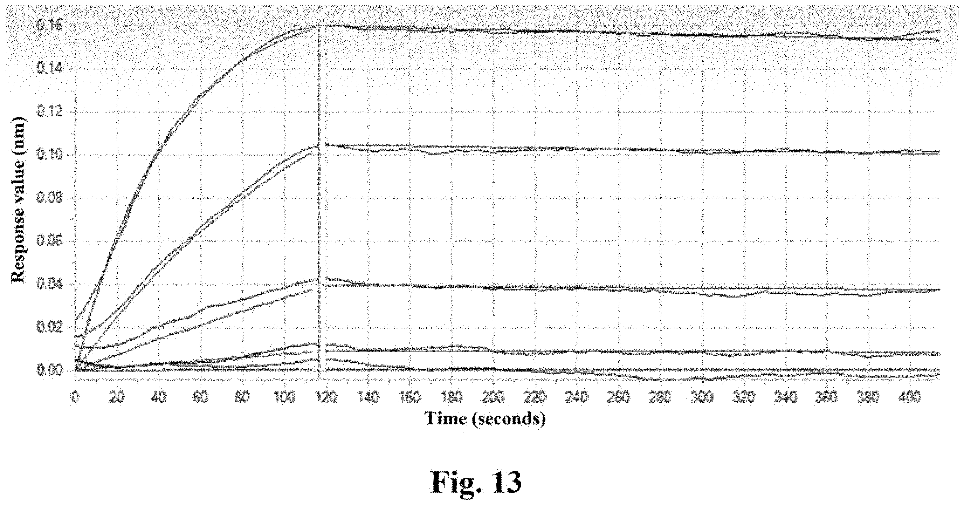

The results of assays for the affinity constants of the humanized antibodies (as control antibodies) for TIGIT are shown in Table 5 and FIGs. 5-13.

**Table 5: The results of assays for the affinity constants of humanized antibodies for antigen TIGIT-mFc**

| Antibody | Maximum signal height of analyte (nm) | K_{D} (M) | kon(1/Ms) | S E (kon) | kdis(1/s) | S E(kdis) | Rmax (nm) |
|---|---|---|---|---|---|---|---|
| 26B12H3L3 | 0.09 | 9.64E-11 | 2.48E+06 | 3.22E+05 | 2.39E-04 | 8.57E-05 | 0.05-0.13 |
| 26B12H1L1 | 0.15 | 1.64E-11 | 5.44E+06 | 1.92E+05 | 8.93E-05 | 3.36E-05 | 0.06-0.17 |
| 26B12H2L2 | 0.14 | 8.40E-12 | 5.47E+06 | 2.55E+05 | 4.60E-05 | 4.40E-05 | 0.07-0.17 |
| 26B12H2L3 | 0.12 | 4.85E-11 | 3.29E+06 | 2.53E+05 | 1.59E-04 | 5.92E-05 | 0.15-0.31 |
| 26B12H3L2 | 0.12 | 5.40E-11 | 3.94E+06 | 3.60E+05 | 2.13E-04 | 7.86E-05 | 0.13-0.17 |
| 26B12H4L4 | 0.13 | 3.69E-11 | 2.81E+06 | 1.38E+05 | 1.04E-04 | 3.23E-05 | 0.14-0.20 |
| 26B12H1L4 | 0.12 | 4.63E-11 | 2.94E+06 | 1.96E+05 | 1.36E-04 | 4.88E-05 | 0.01-0.15 |
| 26B12H4L1 | 0.12 | 8.57E-12 | 2.90E+06 | 1.34E+05 | 2.48E-05 | 3.11E-05 | 0.11-0.18 |
| RG6058 | 0.16 | 3.16E-11 | 4.56E+06 | 1.84E+05 | 1.44E-04 | 3.46E-05 | 0.01-0.18 |

| | | | | | | | |
|---|---|---|---|---|---|---|---|
| K_{D} is the affinity constant; K_{D} = kdis/kon. | | | | | | | |

The results show that the affinity constants of humanized antibodies 26B12H3L3, 26B12H1L1, 26B12H2L2, 26B12H2L3, 26B12H3L2, 26B12H4L4, 26B12H1L4, 26B12H4L1 and RG6058 for TIGIT-mFc were 9.64E-11 M, 1.64E-11 M, 8.40E-12 M, 4.85E-11 M, 5.40E-11 M, 3.69E-11 M, 4.63E-11 M, 8.57E-12 M and 3.16E-11 M, respectively.

The results demonstrate that according to the level of affinity for TIGIT-mFc, the antibodies against TIGIT can be listed in descending order as follows: 26B12H2L2, 26B12H4L1, 26B12H1L1, RG6058, 26B12H4L4, 26B12H1L4, 26B12H2L3, 26B12H3L2, and 26B12H3L3. Among them, the humanized antibodies 26B12H2L2, 26B12H4L1, and 26B12H1L1 have stronger affinity than the positive drug RG6058, and 26B12H4L4 has a comparable affinity to the positive drug RG6058.

### Example 7: FACS assays for activity of humanized antibodies 26B12H2L2 and RG6058 binding to 293T-TIGIT cell membrane surface antigen TIGIT

### Procedures:

TIGIT vector plenti6.3-TIGITFL-BSD (TIGIT is GenbankID: NP_776160.2; human TIGIT full-length cDNA sequence was synthesized by GenScript, designated as TIGITFL, and cloned into pUC57simple vector (supplied by GenScript) to give pUC57simple-TIGITFL plasmid. pUC57simple-TIGITFL plasmid was synthesized with BamHI and XhoI dual enzyme digestion; TIGITFL target gene fragment was collected and subcloned into plenti6.3 expression vector through restriction sites BamHI and XhoI; the vector pLenti6.3 was purchased from Invitrogen) was used to transfect 293T cells, and the cell line 293T-TIGIT stably expressing TIGIT was obtained by screening.

293T-TIGIT cells were collected (DMEM + 10% FBS) and centrifuged for 5 min, and the supernatant was discarded. The cells were resuspended and counted, and the cell viability was determined (P7, 95.79%). The cells were diluted, 300 thousand cells were added to each well of a transparent V-bottomed 96-well plate, and 200 µL of 1% PBSA was added to each tube. The mixtures were centrifuged for 5 min, and the supernatants were discarded. According to the experimental design, 100 µL of antibodies were added to each well (final concentration: 300 nM, 100 nM, 33.3 nM, 11.11 nM, 3.7 nM, 1.23 nM, 0.41 nM, 0.041 nM, and 0.0041 nM), and blank control and isotype control were designed. The plate was incubated on ice for 60 min. 200 µL of 1% PBSA was added to each tube. The mixtures were centrifuged for 5 min, and the supernatants were discarded. The plate was washed twice. FITC goat anti-human IgG antibody (purchased from Jackson, Cat. No. 109-095-098, diluted 500-fold with PBSA) was added to each sample, and the mixtures were incubated on ice in the dark for 40 min. 200 µL of PBSA was added to each tube. The mixtures were centrifuged for 5 min, and the supernatants were discarded. The cells were resuspended by adding 200 µL of PBSA, and the suspensions were transferred into flow cytometry tubes to measure the mean fluorescence intensity of the cells at each concentration by a flow cytometer.

**Table 6: FACS assays for the activity of humanized antibodies 26B12H2L2 and RG6058 binding to 293T-TIGIT cell membrane surface antigen TIGIT**

| Antibody/ concentration (nM) | Mean fluorescence intensity | | | | | | | | | EC₅₀ (nM) |
|---|---|---|---|---|---|---|---|---|---|---|
| | 300 | 100 | 33.3 | 11.11 | 3.7 | 1.23 | 0.41 | 0.041 | 0.0041 | |
| RG6058 | 505.61 | 554.87 | 493.98 | 537.75 | 431.36 | 266.73 | 109.14 | 24.47 | 14.61 | 1.257 |
| 26B12H2L2 | 514.58 | 467.29 | 412.32 | 645.99 | 466.99 | 320.40 | 122.58 | 28.76 | 12.66 | 0.917 |

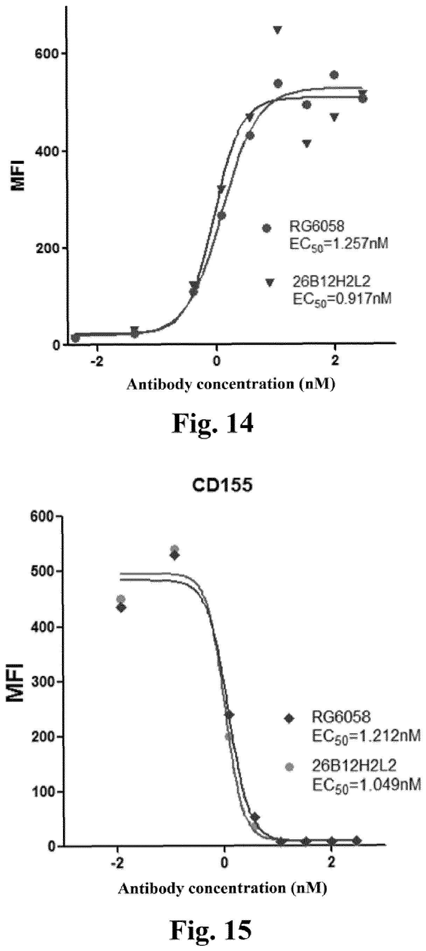

The experimental results are shown in Table 6 and FIG. 14. The EC₅₀ for binding of the positive control antibody RG6058 to the cell membrane surface antigen TIGIT was 1.257 nM, and the EC₅₀ for binding of the humanized antibody 26B12H2L2 to the cell membrane surface antigen TIGIT was 0.917 nM.

The experimental results demonstrate that the humanized antibody 26B12H2L2 has a stronger binding ability to the cell membrane surface antigen TIGIT than the positive control antibody RG6058.

### Example 8: FACS assays for activity of humanized antibodies 26B12H2L2 and RG6058 competing with CD155 or CD112 for binding to 293T-TIGIT cell membrane surface antigen TIGIT

Procedures: 293T-TIGIT cells were collected and centrifuged for 5 min, and the supernatant was discarded. The cells were resuspended and counted, and the cell viability was determined (94.95%). The cells were diluted, 300 thousand cells were added to each well of a transparent V-bottomed 96-well plate, and 200 µL of 1% PBSA was added to each tube. The mixtures were centrifuged for 5 min, and the supernatants were discarded. According to the experimental design, 100 µL of antibodies were added to each well (final concentration: 300 nM, 100 nM, 33.3 nM, 11.1 nM, 3.7 nM, 1.23 nM, 0.123 nM, and 0.0123 nM), and blank control and isotype control were designed. The plate was incubated on ice for 30 min. CD155 (final concentration: 10 nM; produced by Akeso Biopharma, Inc., lot no. 20190726, wherein CD155 was GenBank accession no. NP_006496.4) or CD112 (final concentration: 30 nM; produced by Akeso Biopharma, Inc., lot no. 20190726, wherein CD112 was GenBank accession no. NP_001036189.1) was added to each sample, and the mixtures were incubated on ice in the dark for 60 min. 200 µL of 1% PBSA was then added to each tube. The mixtures were centrifuged for 5 min, and the supernatants were discarded. The plate was washed twice. APC goat anti-mouse IgG antibody (purchased from Biolegend, lot no. 405308, minimal x-reactivity; diluted 300-fold with PBSA) was added to each sample, and the mixtures were incubated on ice in the dark for 40 min. 200 µL of PBSA was added to each tube. The mixtures were centrifuged for 5 min, and the supernatants were discarded. The cells were resuspended by adding 200 µL of PBSA, and the suspensions were transferred into flow cytometry tubes to measure the mean fluorescence intensity of the cells at each concentration by a flow cytometer.

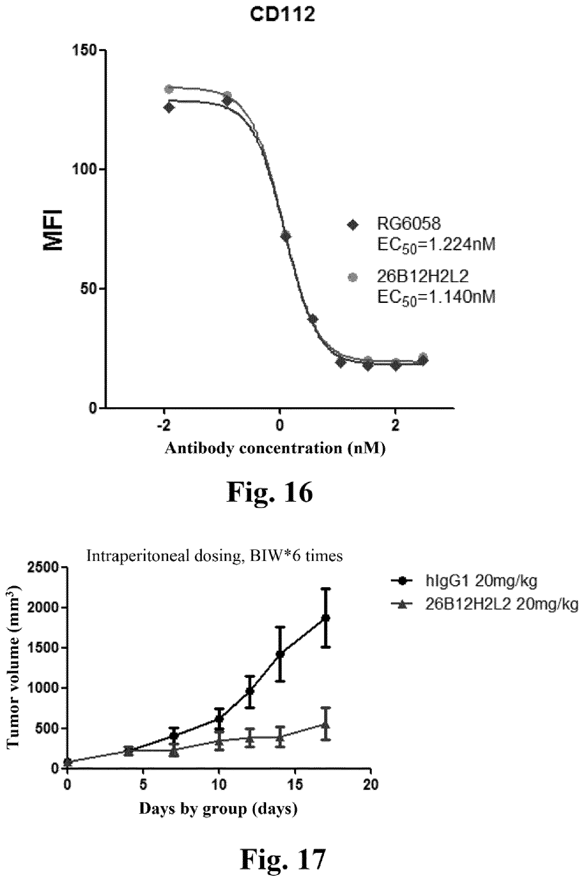

The experimental results are shown in Table 7 and FIG. 15, as well as Table 8 and FIG. 16.

**Table 7: FACS assays for the activity of humanized antibodies 26B12H2L2 and RG6058 competing with CD155 for binding to 293T-TIGIT cell membrane surface antigen TIGIT**

| Antibody/ concentration (nM) | Mean fluorescence intensity | | | | | | | | EC₅₀ (nM) |
|---|---|---|---|---|---|---|---|---|---|
| | 300 | 100 | 33.3 | 11.1 | 3.7 | 1.23 | 0.123 | 0.0123 | |
| RG6058 | 8.85 | 7.44 | 7.6 | 7.71 | 52.1 | 239 | 530 | 436 | 1.212 |
| 26B12H2L2 | 8.3 | 7.64 | 7.83 | 8.2 | 36.1 | 200 | 541 | 449 | 1.049 |

**Table 8: FACS assays for the activity of humanized antibodies 26B12H2L2 and RG6058 competing with CD112 for binding to 293T-TIGIT cell membrane surface antigen TIGIT**

| Antibody/ concentration (nM) | Mean fluorescence intensity | | | | | | | | EC₅₀ (nM) |
|---|---|---|---|---|---|---|---|---|---|
| | 300 | 100 | 33.3 | 11.1 | 3.7 | 1.23 | 0.123 | 0.0123 | |
| RG6058 | 20.1 | 17.8 | 18 | 19.2 | 37.1 | 72 | 129 | 126 | 1.224 |
| 26B12H2L2 | 21.4 | 19.2 | 20.3 | 19.8 | 37.1 | 73.1 | 131 | 134 | 1.140 |

The results show that the EC₅₀ of the positive control antibody RG6058 competing with CD155 for binding to TIGIT was 1.212 nM, and the EC₅₀ of the humanized antibody 26B12H2L2 competing with CD155 for binding to TIGIT was 1.049 nM; the EC₅₀ of the positive control antibody RG6058 competing with CD112 for binding to TIGIT was 1.224 nM, and the EC₅₀ of the humanized antibody 26B12H2L2 competing with CD112 for binding to TIGIT was 1.140 nM.

The experimental results demonstrate that the humanized antibody 26B12H2L2 has a stronger ability to compete with CD155 or CD112 for binding to the cell membrane surface antigen TIGIT than the positive control antibody RG6058.

### Example 9: Therapeutic effect of 26B12H2L2 on CT26 mouse graft tumors in hTigit-BALB/c transgenic mice

hTigit-BALB/c transgenic mice (purchased from Gempharmatech Co., Ltd, in which the normal mouse TIGIT gene was replaced by the human TIGIT gene) were each inoculated with 500 thousand CT26 cells (mouse colon cancer cell line, purchased from ATCC) in the back. Specifically, a mouse tumor model was established by inoculating 200 µL of 25 million/mL CT26 cells into each mouse. The mice were divided into groups of 8 mice, including an isotype control group (20 mg/kg, i.p., twice weekly) and a treatment group (20 mg/kg, i.p., twice weekly). The specific regimen is shown in Table 9.

**Table 9: Establishment of mouse CT26 tumor model and administration regimen of antibodies**

| Group | Number of cells | Number of animals | Modeling | Regimen |
|---|---|---|---|---|
| Isotype control | 500 thousand cells/mouse | 8 | CT26 cells: 25 million cells/mL | **hIgG1** (Akeso Biopharma, Inc., lot no. 20190410) |
| | | | | 20 mg/kg, |
| | | | | Intraperitoneal injection (i.p.), twice weekly |
| 26B12H2L2 | 500 thousand cells/mouse | 8 | Inoculation volume: 200 µL/mouse | 26B12H2L2 20 mg/kg, intraperitoneal injection (i.p.), twice weekly |

The results are shown in FIG. 17.

The results show that 26B12H2L2 caused significant reductions in tumor volume in the hTIGIT-BALB/c transgenic mouse CT26 tumor model.

The results demonstrate that 26B12H2L2 has potent efficacy on the hTIGIT-BALB/c transgenic mouse CT26 tumor model, thus having the potential to treat and/or prevent tumors, particularly colon cancer.

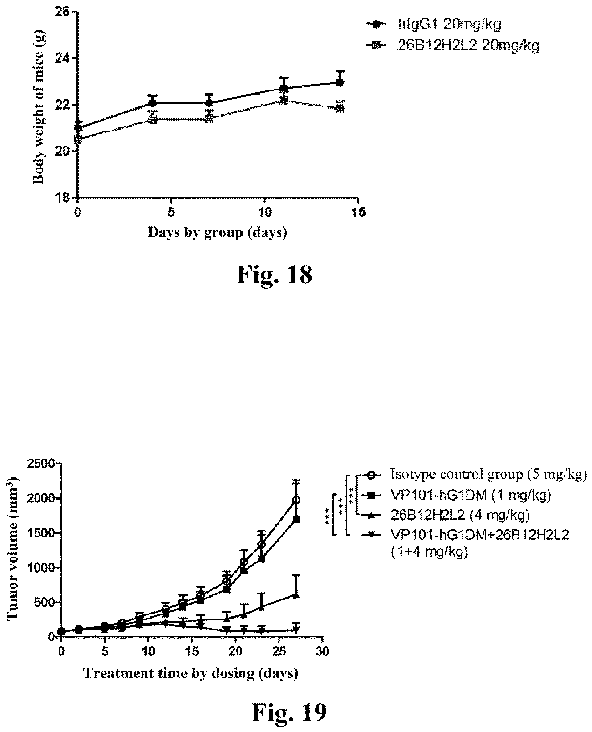

Meanwhile, as shown in FIG. 18, 26B12H2L2 had no effect on the body weight of the hTIGIT-BALB/c transgenic mice as the CT26 tumor model, indicating that the antibody 26B12H2L2 did not cause adverse effects on the mice.

### Example 10: Effective anti-tumor treatment with anti-TIGIT antibody in combination with anti-PD-1/anti-VEGFA bifunctional antibody

In order to determine the anti-tumor activity *in vivo* of the anti-TIGIT antibody in combination with the anti-PD-1/anti-VEGFA bifunctional antibody VP101(hG1DM), CT26 cells (human colon cancer cells, purchased from GemPharmatech Co., Ltd.) were first inoculated subcutaneously into female BALB/c-hPD1/hTIGIT mice aged 5-7 weeks (purchased from GemPharmatech Co., Ltd.), and when the mean tumor volume reached 80-120 mm³, the mice were randomly divided into 4 groups of 6 mice per group based on tumor volume. The day of grouping was defined as D0, and dosing was started on the day of grouping D0. The regimen of the combination therapy group is as follows: the drugs were formulated separately and administered sequentially (no certain order or time interval was required, and one treatment should be given after the completed administration of the other). The modeling and specific regimen are shown in Table 10. After the administration, the length and width of tumors in each group were measured, and the tumor volume was calculated.

**Table 10: Dose regimen of anti-TIGIT antibody in combination with anti-PD-1/anti-VEGFA bifunctional antibody for treating CT26 graft tumor in BALB/c-hPD1/hTIGIT mouse model**

| Grouping | n | Tumor xenograft | Condition of administration |
|---|---|---|---|
| Isotype control 5 mg/kg | 6 | CT26, 5 × 10⁵ cells, BALB/c-hPD 1/hTIGIT mice, SC | Isotype control antibody hlgGl (Akeso Biopharma, Inc., lot no. 20200707), 5 mg/kg; |
| | | | Intraperitoneally administered twice weekly for 4 weeks |
| VP101(hG1DM) | 6 | | VP101(hG1DM), 1 mg/kg; |
| 1mg/kg | | | Intraperitoneally administered twice weekly for 4 weeks |
| 26B12H2L2 | 6 | | 26B 12H2L2, 4 mg/kg; |
| 4mg/kg | | | Intraperitoneally administered twice weekly for 4 weeks |
| 26B12H2L2 | 6 | | 26B12H2L2, 4 mg/kg; VP101(hG1DM), 1 mg/kg; |
| 4mg/kg,VP101(hG1DM) | | | Intraperitoneally administered twice weekly for 4 weeks |
| 1mg/kg | | | |

The results are shown in FIG. 19. The results show that: compared with the isotype control antibody hIgG, both VP101(hG1DM) and 26B12H2L2 can effectively inhibit the growth of mouse tumors. The VP101(hG1DM) + 26B12H2L2 group exhibited synergistic anti-tumor effects on the model, and the combination therapy showed anti-tumor inhibition superior to that of the monotherapies.
*p < 0.05, **p < 0.01, ***p < 0.0001, Two-way *ANOVA* (Bnoferroni posttest)

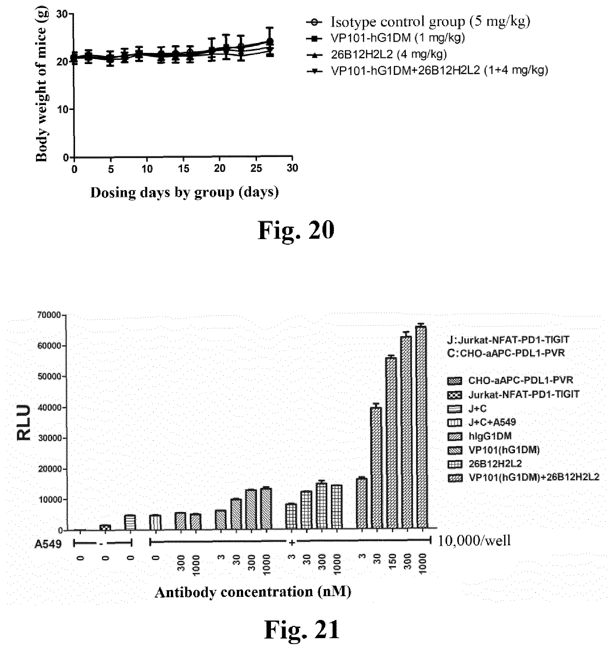

In addition, as shown in FIG. 20, both VP101(hG1DM) and 26B12H2L2 were well tolerated by the tumor-bearing mice, either alone or in combination, and no effect on the body weight of the tumor-bearing mice was found in the groups.

### Example 11: Anti-TIGIT antibody in combination with anti-PD-1/anti-VEGFA bifunctional antibody effectively neutralizing the inhibition of the signaling pathways mediated by the binding of the corresponding targets to the receptors

CHO-aAPC-PDL1-PVR cells (constructed by Akeso Biopharma, Inc.) were collected and centrifuged at 170× g for 5 min before the supernatant was discarded. The cells were resuspended in a complete medium (Ham's F-12 + 10% FBS) and counted, and the viability was calculated. The CHO-aAPC-PDL1-PVR cells were added into a 96-well black plate at 4 × 10⁴/well (100 µL/well), and 150 µL of 1× PBS was added into the edge wells. The plate was incubated overnight in an incubator at 37 °C. Jurkat-NFAT-PD1-TIGIT cells (constructed by Akeso Biopharma, Inc.) were collected and centrifuged at 110× g for 5 min before the supernatant was discarded. The cells were resuspended in an analysis culture medium (RPMI 1640 + 10% FBS) and counted, and the viability was calculated. The liquid in the 96-well black plate was discarded, Jurkat-NFAT-PD1-TIGIT cells were added at 30 µL/well (5 × 10⁴/well); A549 lung cancer cells (purchased from Chinese Academy of Sciences, Shanghai Institutes for Biological Sciences Cell Center, Cat. No. SCSP-503) were collected and centrifuged at 170× g for 5 min before the supernatant was discarded. The cells were resuspended in an analysis culture medium and counted, and the viability was calculated. A549 cells were added at 30 µL/well (1 × 10⁴/well). Antibodies were diluted according to the design (the final concentrations of VP101(hG1DM) and 26B12H2L2 in monotherapy groups were 3 nM, 30 nM, 300 nM, and 1000 nM, and the final concentrations of VP101(hG1DM) and 26B12H2L2 in the VP101(hG1DM) + 26B12H2L2 combination groups were 3 nM, 30 nM, 150 nM, 300 nM, and 1000 nM), and added at 20 µL/well. An isotype control group and a negative control group were set with a final volume of 80 µL/well. The cells were incubated for 6 h in an incubator at 37 °C. After 6 hours, 80 µL of the reaction solution of the Firefly Glo Luciferase Reporter Gene Assay Kit (purchased from Yeasen, Cat. No. 11404ES80) was added, and after 5 to 6 minutes of incubation, the plates were loaded on an apparatus for detecting the relative light unit (RLU).

The results are shown in FIG. 21.

The results show that: compared with the isotype control antibody hIgG1DM, the monotherapy VP101(hG1DM), the monotherapy 26B12H2L2 and the VP101(hG1DM) + 26B12H2L2 combination therapy can effectively neutralize the inhibition effect on signaling pathways mediated by the binding of the corresponding targets to receptors, and enhance the expression of luciferase. Among these, the RLU values of 150 nM and 300 nM combination groups were significantly higher than those of 300 nM and 1000 nM monotherapy groups (> 3 times), suggesting that the neutralizing activity of VP101(hG1DM) + 26B12H2L2 combination therapy is significantly better than those of the monotherapies.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

### SEQUENCE LISTING (Note: those underlined are CDR sequences)

Amino acid sequence of 26B12VH 
EVQLQESGPGLVKPSQSLSLTCTVTGHSFTSDYAWNWIRQFPGNRLEWMG
YISYSDSTNYNPSLKSRISITRDTSKNQFFLQMNSVTTEDTATYYCARLDYG
NYGGAMDYWGQGTSVTVSS (SEQ ID NO: 1)
Nucleotide sequence of 26B12VH 
GAGGTGCAGCTGCAGGAGTCTGGACCTGGCCTGGTGAAACCCTCTCAGT
CTCTGTCCCTCACCTGCACTGTCACTGGCCACTCATTCACCAGTGATTAT
GCCTGGAACTGGATCCGGCAGTTTCCAGGAAACAGACTGGAGTGGATG
GGCTACATAAGCTACAGTGATAGCACTAACTACAACCCATCTCTCAAAA
GTCGAATCTCTATCACTCGAGACACATCCAAGAACCAGTTCTTCTTGCA
GATGAATTCTGTGACTACTGAGGACACAGCCACATATTACTGTGCAAGA
TTGGACTATGGTAACTACGGTGGGGCTATGGACTACTGGGGTCAAGGG
ACCTCAGTCACCGTCTCCTCA (SEQ ID NO: 2)
26B12VH HCDR1: GHSFTSDYA (SEQ ID NO: 3)
26B12VH HCDR2: ISYSDST (SEQ ID NO: 4)
26B12VH HCDR3: ARLDYGNYGGAMDY (SEQ ID NO: 5)
Amino acid sequence of 26B12VL 
DIVLTQSHEFMSTSLRDRVSITCKSSQHVSTAVAWYQQKPGQSPKLLIYSAS
YRYTGVPDRFTGSGSGTDFTFTISSVKAEDLAVYYCQQHYITPWTFGGGTK
 LEIK (SEQ ID NO: 6)
Nucleotide sequence of 26B12VL 
GATATTGTGCTAACTCAGTCTCACGAATTCATGTCCACCTCATTACGAG
ACAGGGTCAGCATCACCTGCAAATCCAGTCAACATGTGAGTACTGCTGT
AGCCTGGTATCAACAGAAACCAGGACAATCTCCTAAACTACTGATTTAC
TCGGCATCCTACCGGTACACTGGAGTCCCTGATCGCTTCACTGGCAGTG
GATCTGGGACGGATTTCACTTTCACCATCAGCAGTGTGAAGGCTGAAGA
CCTGGCAGTTTATTACTGTCAGCAACATTATATTACTCCGTGGACGTTCG
GTGGAGGCACCAAGCTGGAAATAAAA (SEQ ID NO: 7)
26B12VL LCDR1: QHVSTA (SEQ ID NO: 8)
26B12VL LCDR2: SAS (SEQ ID NO: 9)
26B12VL LCDR3: QQHYITPWT (SEQ ID NO: 10)
Amino acid sequence of 26B12H1 
DVQLQESGPGLVKPSQTLSLTCTVSGHSFTSDYAWNWIRQFPGKGLEWIGY
ISYSDSTNYNPSLKSRITISRDTSKNQFFLQLNSVTAADTATYYCARLDYGN
YGGAMDYWGQGTSVTVSS (SEQ ID NO: 11)
Nucleotide sequence of 26B12H1 
GATGTGCAGCTGCAGGAGAGCGGCCCCGGACTGGTGAAGCCTTCCCAG
ACCCTGTCTCTGACCTGTACAGTGTCTGGCCACAGCTTCACATCCGACT
ACGCCTGGAACTGGATCAGGCAGTTTCCAGGCAAGGGCCTGGAGTGGA
TCGGCTACATCTCTTATAGCGACTCCACCAACTATAATCCCTCTCTGAA
GAGCCGGATCACCATCAGCAGAGATACATCCAAGAACCAGTTCTTTCTG
CAGCTGAACAGCGTGACAGCCGCCGACACCGCCACATACTATTGCGCC
 
CGGCTGGACTACGGCAATTATGGCGGAGCCATGGATTACTGGGGCCAG
GGCACCTCCGTGACAGTGAGCTCC (SEQ ID NO: 12)
Amino acid sequence of 26B12H2 
DVQLQESGPGLVKPSQTLSLTCTVSGHSFTSDYAWSWIRQPPGKGLEWIGY
ISYSDSTNYNPSLKSRVTISRDTSKNQFSLKLSSVTAADTAVYYCARLDYGN
YGGAMDYWGQGTSVTVSS (SEQ ID NO: 13)
Nucleotide sequence of 26B12H2 
GATGTGCAGCTGCAGGAGTCTGGCCCAGGACTGGTGAAGCCAAGCCAG
ACCCTGTCCCTGACCTGTACAGTGTCCGGCCACTCTTTTACAAGCGACT
ACGCCTGGTCTTGGATCAGGCAGCCCCCTGGCAAGGGACTGGAGTGGA
TCGGCTACATCTCCTATTCTGACAGCACCAACTATAATCCCTCCCTGAA
GTCTCGGGTGACCATCTCTAGAGATACAAGCAAGAACCAGTTCTCCCTG
AAGCTGAGCTCCGTGACCGCAGCAGACACAGCCGTGTACTATTGCGCCC
GGCTGGACTACGGCAATTATGGCGGAGCCATGGATTACTGGGGCCAGG
GCACCAGCGTGACAGTGTCTAGC (SEQ ID NO: 14)
Amino acid sequence of 26B12H3 
DVQLQESGPGLVKPSQTLSLTCTVSGHSFTSDYAWSWIRQPPGKGLEWIGY
ISYSDSTNYNPSLKSRVTISVDTSKNQFSLKLSSVTAADTAVYYCARLDYG
NYGGAMDYWGQGTSVTVSS (SEQ ID NO: 15)
Nucleotide sequence of 26B12H3 
GATGTGCAGCTGCAGGAGTCTGGCCCAGGACTGGTGAAGCCAAGCCAG
ACCCTGTCCCTGACCTGTACAGTGTCCGGCCACTCTTTACAAGCGACT
ACGCCTGGTCTTGGATCAGACAGCCCCCTGGCAAGGGACTGGAGTGGA
 
TCGGCTACATCTCCTATTCTGACAGCACCAACTATAATCCCTCCCTGAA
GTCTAGAGTGACCATCTCTGTGGATACAAGCAAGAACCAGTTCTCCCTG
AAGCTGAGCTCCGTGACCGCAGCAGACACAGCCGTGTACTATTGCGCCC
GGCTGGACTACGGCAATTATGGCGGAGCCATGGATTACTGGGGCCAGG
GCACCAGCGTGACAGTGTCTAGC (SEQ ID NO: 16)
Amino acid sequence of 26B12H4 
DVQLQESGPGLVKPSQTLSLTCTVSGHSFTSDYAWNWIRQFPGKGLEWMG
YISYSDSTNYNPSLKSRITISRDTSKNQFFLQLNSVTAADTATYYCARLDYG
NYGGAMDYWGQGTSVTVSS (SEQ ID NO: 17)
Nucleotide sequence of 26B12H4 
GATGTGCAGCTGCAGGAGAGCGGCCCCGGACTGGTGAAGCCTTCCCAG
ACCCTGTCTCTGACCTGTACAGTGTCTGGCCACAGCTTCACATCCGACT
ACGCCTGGAACTGGATCAGGCAGTTTCCAGGCAAGGGCCTGGAGTGGA
TGGGCTACATCTCTTATAGCGACTCCACCAACTATAATCCCTCTCTGAA
GAGCCGGATCACCATCAGCAGAGATACATCCAAGAACCAGTTCTTTCTG
CAGCTGAACAGCGTGACAGCCGCCGACACCGCCACATACTATTGCGCC
CGGCTGGACTACGGCAATTATGGCGGAGCCATGGATTACTGGGGCCAG
GGCACCTCCGTGACAGTGAGCTCC (SEQ ID NO: 18)
Amino acid sequence of 26B12L1 
DIQMTQSPKSLSTSVGDRVTITCRSSQHVSTAVAWYQQKPGKSPKLLIYSAS
YRYSGVPDRFSGSGSGTDFTFTISSVQPEDFATYYCQQHYITPWTFGGGTKL
EIK (SEQ ID NO: 19)
Nucleotide sequence of 26B12L1 
GACATCCAGATGACCCAGTCCCCTAAGTCCCTGTCTACAAGCGTGGGCG
ATCGGGTGACCATCACATGTAGAAGCTCCCAGCACGTGTCTACCGCAGT
GGCATGGTACCAGCAGAAGCCAGGCAAGAGCCCTAAGCTGCTGATCTA
TTCCGCCTCTTACAGGTATTCCGGAGTGCCAGACCGGTTTAGCGGCTCC
GGCTCTGGCACCGATTTCACCTTTACAATCTCTAGCGTGCAGCCAGAGG
ACTTCGCCACATACTATTGCCAGCAGCACTACATCACCCCATGGACCTT
CGGCGGCGGCACAAAGCTGGAGATCAAG (SEQ ID NO: 20)
Amino acid sequence of 26B12L2 
DIQMTQSPSSLSASVGDRVTITCRSSQHVSTALAWYQQKPGKSPKLLIYSAS
SRYSGVPDRFSGSGSGTDFTFTISSLQPEDFATYYCQQHYITPWTFGGGTKL
EIK (SEQ ID NO: 21)
Nucleotide sequence of 26B12L2 
GACATCCAGATGACCCAGTCCCCTAGCTCCCTGTCTGCCAGCGTGGGCG
ATAGGGTGACCATCACATGTAGATCTAGCCAGCACGTGTCTACAGCCCT
GGCATGGTACCAGCAGAAGCCAGGCAAGAGCCCTAAGCTGCTGATCTA
CTCCGCCTCCTCTAGGTATTCTGGAGTGCCAGACCGGTTTTCCGGCTCTG
GCAGCGGCACCGATTTCACCTTTACAATCAGCTCCCTGCAGCCAGAGGA
CTTCGCCACATACTATTGCCAGCAGCACTATATCACCCCATGGACCTTC
GGCGGCGGCACCAAGCTGGAGATCAAG (SEQ ID NO: 22)
Amino acid sequence of 26B12L3 
DIQMTQSPSSLSASVGDRVTITCRASQHVSTALAWYQQKPGKAPKLLIYSA
SSLQSGVPSRFSGSGSGTDFTLTISSLQPEDFATYYCQQHYITPWTFGGGTK
LEIK (SEQ ID NO: 23)
Nucleotide sequence of 26B12L3 
GACATCCAGATGACCCAGTCCCCTAGCTCCCTGAGCGCCTCCGTGGGCG
ATAGGGTGACCATCACATGTAGAGCCTCTCAGCACGTGAGCACAGCCCT
GGCATGGTACCAGCAGAAGCCAGGCAAGGCCCCTAAGCTGCTGATCTA
TAGCGCCTCTAGCCTGCAGTCCGGAGTGCCATCTCGGTTCTCTGGCAGC
GGCTCCGGAACCGACTTTACCCTGACAATCTCCTCTCTGCAGCCAGAGG
ATTTCGCCACATACTATTGCCAGCAGCACTACATCACCCCATGGACCTT
CGGCGGCGGCACCAAGCTGGAGATCAAG (SEQ ID NO: 24)
Amino acid sequence of 26B12L4 
DIQMTQSPKSMSTSVGDRVTITCRSSQHVSTAVAWYQQKPGKSPKLLIYSA
SYRYSGVPDRFSGSGSGTDFTFTISSVQPEDFATYYCQQHYITPWTFGGGTK
LEIK (SEQ ID NO: 25)
Nucleotide sequence of 26B12L4 
GACATCCAGATGACCCAGTCCCCTAAGTCCATGTCTACAAGCGTGGGCG
ACAGGGTGACCATCACATGTAGAAGCTCCCAGCACGTGTCTACCGCAGT
GGCATGGTACCAGCAGAAGCCAGGCAAGAGCCCTAAGCTGCTGATCTA
TTCCGCCTCTTACAGGTATTCCGGAGTGCCAGACCGGTTTAGCGGCTCC
GGCTCTGGCACCGATTTCACCTTTACAATCTCTAGCGTGCAGCCAGAGG
ACTTCGCCACATACTATTGCCAGCAGCACTACATCACCCCATGGACCTT
CGGCGGCGGCACAAAGCTGGAGATCAAG (SEQ ID NO: 26)
Amino acid sequence of VP101(hG1DM) heavy chain 
EVQLVESGGGLVQPGGSLRLSCAASGYTFTNYGMNWVRQAPGKGLEWVG
WINTYTGEPTYAADFKRRFTFSLDTSKSTAYLQMNSLRAEDTAVYYCAKY
PHYYGSSHWYFDVWGQGTLVTVSSASTKGPSVFPLAPSSKSTSGGTAALG
 
CLVKDYFPEPVTVSWNSGALTSGVHTFPAVLQSSGLYSLSSVVTVPSSSLGT
QTYICNVNHKPSNTKVDKKVEPKSCDKTHTCPPCPAPEAAGGPSVFLFPPK
PKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGK
GGGGSGGGGSGGGGSGGGGSEVQLVESGGGLVQPGGSLRLSCAASGFAFS
SYDMSWVRQAPGKGLDWVATISGGGRYTYYPDSVKGRFTISRDNSKNNL
YLQMNSLRAEDTALYYCANRYGEAWFAYWGQGTLVTVSSGGGGSGGGG
SGGGGSGGGGSDIQMTQSPSSMSASVGDRVTFTCRASQDINTYLSWFQQKP
GKSPKTLIYRANRLVSGVPSRFSGSGSGQDYTLTISSLQPEDMATYYCLQY
DEFPLTFGAGTKLELKR (SEQ ID NO: 27)
Nucleotide sequence of VP101(hG1DM) heavy chain 
GAGGTGCAGCTGGTCGAGTCCGGGGGGGGCTGGTGCAGCCAGGCGGG
TCTCTGAGGCTGAGTTGCGCCGCTTCAGGGTACACCTTCACAAACTATG
GAATGAATTGGGTGCGCCAGGCACCAGGAAAGGGACTGGAGTGGGTCG
GCTGGATCAACACTTACACCGGGGAACCTACCTATGCAGCCGACTTTAA
GCGGCGGTTCACCTTCAGCCTGGATACAAGCAAATCCACTGCCTACCTG
CAGATGAACAGCCTGCGAGCTGAGGACACCGCAGTCTACTATTGTGCTA
AATATCCCCACTACTATGGGAGCAGCCATTGGTATTTTGACGTGTGGG
GCAGGGGACTCTGGTGACAGTGAGCAGCGCAAGCACCAAAGGGCCCAG
CGTGTTTCCTCTCGCCCCCTCCTCCAAAAGCACCAGCGGAGGAACCGCT
GCTCTCGGATGTCTGGTGAAGGACTACTTCCCTGAACCCGTCACCGTGA
GCTGGAATAGCGGCGCTCTGACAAGCGGAGTCCATACATTCCCTGCTGT
GCTGCAAAGCAGCGGACTCTATTCCCTGTCCAGCGTCGTCACAGTGCCC
AGCAGCAGCCTGGGCACCCAGACCTACATCTGTAACGTCAACCACAAG
 
CCCTCCAACACCAAGGTGGACAAGAAAGTGGAGCCCAAATCCTGCGAC
AAGACACACACCTGTCCCCCCTGTCCTGCTCCCGAAGCTGCTGGAGGCC
CTAGCGTCTTCCTCTTTCCTCCCAAACCCAAGGACACCCTCATGATCAG
CAGAACCCCTGAAGTCACCTGTGTCGTCGTGGATGTCAGCCATGAGGAC
CCCGAGGTGAAATTCAACTGGTATGTCGATGGCGTCGAGGTGCACAAC
GCCAAAACCAAGCCCAGGGAGGAACAGTACAACTCCACCTACAGGGTG
GTGTCCGTGCTGACAGTCCTCCACCAGGACTGGCTGAACGGCAAGGAG
TACAAGTGCAAGGTGTCCAACAAGGCTCTCCCTGCCCCCATTGAGAAGA
CCATCAGCAAGGCCAAAGGCCAACCCAGGGAGCCCCAGGTCTATACAC
TGCCTCCCTCCAGGGACGAACTCACCAAGAACCAGGTGTCCCTGACCTG
CCTGGTCAAGGGCTTTTATCCCAGCGACATCGCCGTCGAGTGGGAGTCC
AACGGACAGCCCGAGAATAACTACAAGACCACCCCTCCTGTCCTCGACT
CCGACGGCTCCTTCTTCCTGTACAGCAAACTGACCGTCGATAAATCTAG
GTGGCAGCAGGGCAACGTGTTCTCTTGTTCCGTGATGCATGAAGCACTG
CACAACCATTATACCCAGAAGTCTCTGAGCCTGTCCCCCGGCAAGGGCG
GCGGCGGCTCTGGAGGAGGAGGCAGCGGCGGAGGAGGCTCCGGAGGC
GGCGGCTCTGAGGTGCAGCTGGTGGAGTCTGGAGGAGGACTGGTGCAG
CCTGGAGGCTCCCTGAGGCTGTCTTGCGCAGCAAGCGGATTCGCCTTTA
GCTCCTACGACATGAGCTGGGTGCGGCAGGCACCTGGCAAGGGTCTGG
ATTGGGTGGCAACCATCAGCGGAGGCGGCAGATACACATACTATCCCG
ACTCCGTGAAGGGCAGGTTCACCATCTCCCGCGATAACTCTAAGAACAA
TCTGTATCTGCAGATGAACAGCCTGAGGGCCGAGGACACAGCCCTGTA
CTATTGCGCCAACCGCTACGGCGAGGCCTGGTTTGCCTATTGGGGCCAG
GGCACCCTGGTGACAGTGTCTAGCGGCGGCGGCGGCAGCGGCGGCGGC
GGCTCCGGAGGAGGCGGCTCTGGCGGCGGCGGCAGCGATATCCAGATG
ACCCAGTCCCCCTCCTCTATGTCTGCCAGCGTGGGCGACCGGGTGACCT
TCACATGTAGAGCCTCCCAGGATATCAACACCTACCTGTCTTGGTTTCA
 
GCAGAAGCCCGGCAAGAGCCCTAAGACACTGATCTATCGGGCCAATAG
ACTGGTGAGCGGAGTGCCTTCCCGGTTCTCCGGCTCTGGCAGCGGACAG
GACTATACCCTGACAATCAGCTCCCTGCAGCCAGAGGATATGGCCACAT
ACTATTGCCTGCAGTATGACGAGTTCCCCCTGACCTTCGGGGCTGGCAC
TAAGCTGGAGCTGAAAAGA (SEQ ID NO: 28)
Amino acid sequence of VP101(hG1DM) light chain 
DIQMTQSPSSLSASVGDRVTITCSASQDISNYLNWYQQKPGKAPKVLIYFTS
SLHSGVPSRFSGSGSGTDFTLTISSLQPEDFATYYCQQYSTVPWTFGQGTKV
EIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVT
KSFNRGEC (SEQ ID NO: 29)
Nucleotide sequence of VP101(hG1DM) light chain 
GATATTCAGATGACTCAGAGCCCCTCCTCCCTGTCCGCCTCTGTGGGCG
ACAGGGTCACCATCACATGCAGTGCTTCACAGGATATTTCCAACTACCT
GAATTGGTATCAGCAGAAGCCAGGAAAAGCACCCAAGGTGCTGATCTA
CTTCACTAGCTCCCTGCACTCAGGAGTGCCAAGCCGGTTCAGCGGATCC
GGATCTGGAACCGACTTTACTCTGACCATTTCTAGTCTGCAGCCTGAGG
ATTTCGCTACATACTATTGCCAGCAGTATTCTACCGTGCCATGGACATTT
GGCCAGGGGACTAAAGTCGAGATCAAGCGGACCGTGGCCGCTCCCAGT
GTCTTCATTTTCCCCCTAGCGACGAACAGCTGAAATCCGGGACAGCCT
CTGTGGTCTGTCTGCTGAACAACTTCTACCCTAGAGAGGCAAAAGTGCA
GTGGAAGGTCGATAACGCCCTGCAGAGTGGCAATTCACAGGAGAGCGT
GACAGAACAGGACTCCAAAGATTCTACTTATAGTCTGTCAAGCACACTG
ACTCTGAGCAAGGCTGACTACGAAAAGCATAAAGTGTATGCATGTGAG
GTCACCCACCAGGGGCTGAGCAGTCCAGTCACCAAGTCATTCAACAGA
 GGCGAGTGC (SEQ ID NO: 30)
Amino acid sequence of bevacizumab heavy chain variable region (Bevacizumab-Hv): (123 aa) 


 (SEQ ID NO: 31)
Nucleotide sequence of bevacizumab heavy chain variable region: (369 bp) 
GAGGTGCAGCTGGTCGAGTCCGGGGGGGGCTGGTGCAGCCAGGCGGG
TCTCTGAGGCTGAGTTGCGCCGCTTCAGGGTACACCTTCACAAACTATG
GAATGAATTGGGTGCGCCAGGCACCAGGAAAGGGACTGGAGTGGGTCG
GCTGGATCAACACTTACACCGGGGAACCTACCTATGCAGCCGACTTTAA
GCGGCGGTTCACCTTCAGCCTGGATACAAGCAAATCCACTGCCTACCTG
CAGATGAACAGCCTGCGAGCTGAGGACACCGCAGTCTACTATTGTGCTA
AATATCCCCACTACTATGGGAGCAGCCATTGGTATTTTGACGTGTGGGG
GCAGGGGACTCTGGTGACAGTGAGCAGC (SEQ ID NO: 32)
Amino acid sequence of bevacizumab light chain variable region (Bevacizumab-Lv): (107 aa) 


 (SEQ ID NO: 33)
Nucleotide sequence of bevacizumab light chain variable region: (321 bp) 
GATATTCAGATGACTCAGAGCCCCTCCTCCCTGTCCGCCTCTGTGGGCG
ACAGGGTCACCATCACATGCAGTGCTTCACAGGATATTTCCAACTACCT
 
GAATTGGTATCAGCAGAAGCCAGGAAAAGCACCCAAGGTGCTGATCTA
CTTCACTAGCTCCCTGCACTCAGGAGTGCCAAGCCGGTTCAGCGGATCC
GGATCTGGAACCGACTTTACTCTGACCATTTCTAGTCTGCAGCCTGAGG
ATTTCGCTACATACTATTGCCAGCAGTATTCTACCGTGCCATGGACATTT
GGCCAGGGGACTAAAGTCGAGATCAAG (SEQ ID NO: 34)
Bevacizumab heavy chain variable region HCDR1: GYTFTNYG (SEQ ID NO: 35)
Bevacizumab heavy chain variable region HCDR2: INTYTGEP (SEQ ID NO: 36)
Bevacizumab heavy chain variable region HCDR3: AKYPHYYGSSHWYFDV (SEQ ID NO: 37)
Bevacizumab light chain variable region LCDR1: QDISNY (SEQ ID NO: 38)
Bevacizumab light chain variable region LCDR2: FTS (SEQ ID NO: 39)
Bevacizumab light chain variable region LCDR3: QQYSTVPWT (SEQ ID NO: 40)
Amino acid sequence of humanized monoclonal antibody 14C12H1L1 heavy chain variable region: (118 aa) 
EVQLVESGGGLVQPGGSLRLSCAASGFAFSSYDMSWVRQAPGKGLDWVA
TISGGGRYTYYPDSVKGRFTISRDNSKNNLYLQMNSLRAEDTALYYCANR
YGEAWFAYWGQGTLVTVSS (SEQ ID NO: 41)
Nucleotide sequence of humanized monoclonal antibody 14C12H1L1 heavy chain variable region: (354 bp) 
GAAGTGCAGCTGGTCGAGTCTGGGGGAGGGCTGGTGCAGCCCGGCGGG
TCACTGCGACTGAGCTGCGCAGCTTCCGGATTCGCCTTTAGCTCCTACG
ACATGTCCTGGGTGCGACAGGCACCAGGAAAGGGACTGGATTGGGTCG
 
CTACTATCTCAGGAGGCGGGAGATACACCTACTATCCTGACAGCGTCAA
GGGCCGGTTCACAATCTCTAGAGATAACAGTAAGAACAATCTGTATCTG
CAGATGAACAGCCTGAGGGCTGAGGACACCGCACTGTACTATTGTGCC
AACCGCTACGGGGAAGCATGGTTTGCCTATTGGGGGCAGGGAACCCTG
GTGACAGTCTCTAGT (SEQ ID NO: 42)
Amino acid sequence of 14C12H1L1(M) light chain variable region: (108 aa, amino acid mutation sites based on 14C12H1L1 are underlined) 
DIQMTQSPSSMSASVGDRVTFTCRASQDINTYLSWFQQKPGKSPKTLIYRA
NRLVSGVPSRFSGSGSGQDYTLTISSLQPEDMATYYCLQYDEFPLTFGAGT
KLELKR (SEQ ID NO: 43)
Nucleotide sequence of 14C12H1L1(M) light chain variable region: 
GATATCCAGATGACCCAGTCCCCCTCCTCTATGTCTGCCAGCGTGGGCG
ACCGGGTGACCTTCACATGTAGAGCCTCCCAGGATATCAACACCTACCT
GTCTTGGTTTCAGCAGAAGCCCGGCAAGAGCCCTAAGACACTGATCTAT
CGGGCCAATAGACTGGTGAGCGGAGTGCCTTCCCGGTTCTCCGGCTCTG
GCAGCGGACAGGACTATACCCTGACAATCAGCTCCCTGCAGCCAGAGG
ATATGGCCACATACTATTGCCTGCAGTATGACGAGTTCCCCCTGACCTT
CGGGGCTGGCACTAAGCTGGAGCTGAAAAGA (SEQ ID NO: 44)
14C12H1L1(M) heavy chain variable region HCDR1: GFAFSSYD (SEQ ID NO: 45)
14C12H1L1(M) heavy chain variable region HCDR2: ISGGGRYT (SEQ ID NO: 46)
14C12H1L1(M) heavy chain variable region HCDR3: ANRYGEAWFAY (SEQ ID NO: 47)
14C12H1L1(M) light chain variable region LCDR1: QDINTY (SEQ ID NO: 48)
14C12H1L1(M) light chain variable region LCDR2: RAN (SEQ ID NO: 49)
14C12H1L1(M) light chain variable region LCDR3: LQYDEFPLT (SEQ ID NO: 50)
Amino acid sequence of mFc 
PRGPTIKPCPPCKCPAPNLLGGPSVFIFPPKIKDVLMISLSPIVTCVVVDVSED
DPDVQISWFVNNVEVHTAQTQTHREDYNSTLRVVSALPIQHQDWMSGKEF
KCKVNNKDLPAPIERTISKPKGSVRAPQVYVLPPPEEEMTKKQVTLTCMVT
DFMPEDIYVEWTNNGKTELNYKNTEPVLDSDGSYFMYSKLRVEKKNWVE
RNSYSCSVVHEGLHNHHTTKSFSRTPGK (SEQ ID NO: 51)
Amino acid sequence of the first linker fragment
   GGGGSGGGGSGGGGS (SEQ ID NO: 52)
Amino acid sequence of the second linker fragment
   GGGGSGGGGSGGGGSGGGGS (SEQ ID NO: 53)
Amino acid sequence of hFc 
THTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFS
CSVMHEALHNHYTQKSLSLSPGK (SEQ ID NO: 54)
Amino acid sequence of hIgG1DM heavy chain constant region

ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNV
FSCSVMHEALHNHYTQKSLSLSPGK (SEQ ID NO: 55)

## Claims

1. A pharmaceutical composition comprising an anti-TIGIT antibody or an antigen-binding fragment thereof, and an anti-PD-1/anti-VEGFA bispecific antibody or an antigen-binding fragment thereof, wherein optionally, the pharmaceutical composition further comprises a pharmaceutically acceptable carrier and/or excipient,
wherein the anti-TIGIT antibody comprises HCDR1-HCDR3 comprised in a heavy chain variable region set forth in SEQ ID NO: 1 and LCDR1-LCDR3 comprised in a light chain variable region set forth in SEQ ID NO: 6 (preferably, according to the IMGT numbering system, the heavy chain variable region of the antibody comprises HCDR1-HCDR3 having amino acid sequences set forth in SEQ ID NOs: 3-5, respectively, and the light chain variable region of the antibody comprises LCDR1-LCDR3 having amino acid sequences set forth in SEQ ID NOs: 8-10, respectively),
the anti-PD-1/anti-VEGFA bispecific antibody comprises:
a first protein functional region targeting PD-1, and
a second protein functional region targeting VEGFA;
wherein the first protein functional region is an immunoglobulin, and the second protein functional region is a single chain antibody; or, the first protein functional region is a single chain antibody, and the second protein functional region is an immunoglobulin; wherein,
the immunoglobulin comprises HCDR1-HCDR3 comprised in a heavy chain variable region set forth in SEQ ID NO: 31 (preferably, HCDR1-HCDR3 set forth in SEQ ID NOs: 35-37, respectively, according to the IMGT numbering system), and LCDR1-LCDR3 comprised in a light chain variable region set forth in SEQ ID NO: 33 (preferably, LCDR1-LCDR3 set forth in SEQ ID NOs: 38-40, respectively, according to the IMGT numbering system); and
the single chain antibody comprises HCDR1-HCDR3 comprised in a heavy chain variable region set forth in SEQ ID NO: 41 (preferably, HCDR1-HCDR3 set forth in SEQ ID NOs: 45-47, respectively, according to the IMGT numbering system), and LCDR1-LCDR3 comprised in a light chain variable region set forth in SEQ ID NO: 43 (preferably, LCDR1-LCDR3 set forth in SEQ ID NOs: 48-50, respectively, according to the IMGT numbering system);
or,
the immunoglobulin comprises a heavy chain variable region comprising HCDR1-HCDR3 comprised in a heavy chain variable region having an amino acid sequence set forth in SEQ ID NO: 41 (preferably, HCDR1-HCDR3 set forth in SEQ ID NOs: 45-47, respectively, according to the IMGT numbering system), and a light chain variable region comprising LCDR1-LCDR3 comprised in a light chain variable region having an amino acid sequence set forth in SEQ ID NO: 43 (preferably LCDR1-LCDR3 set forth in SEQ ID NOs: 48-50, respectively, according to the IMGT numbering system);
the single chain antibody comprises a heavy chain variable region comprising HCDR1-HCDR3 comprised in a heavy chain variable region having an amino acid sequence set forth in SEQ ID NO: 31 (preferably, HCDR1-HCDR3 set forth in SEQ ID NOs: 35-37, respectively, according to the IMGT numbering system), and a light chain variable region comprising LCDR1-LCDR3 comprised in a light chain variable region having an amino acid sequence set forth in SEQ ID NO: 33 (preferably LCDR1-LCDR3 set forth in SEQ ID NOs: 38-40, respectively, according to the IMGT numbering system);
the immunoglobulin is of human IgG1 subtype.

2. The pharmaceutical composition according to claim 1, wherein the heavy chain variable region of the anti-TIGIT antibody has an amino acid sequence selected from: SEQ ID NO: 1, SEQ ID NO: 11, SEQ ID NO: 13, SEQ ID NO: 15 and SEQ ID NO: 17; the light chain variable region of the anti-TIGIT antibody has an amino acid sequence selected from: SEQ ID NO: 6, SEQ ID NO: 19, SEQ ID NO: 21, SEQ ID NO: 23 and SEQ ID NO: 25;
preferably, for the anti-TIGIT antibody or the antigen-binding fragment thereof, the heavy chain variable region of the antibody has an amino acid sequence set forth in SEQ ID NO: 1, and the light chain variable region of the antibody has an amino acid sequence set forth in SEQ ID NO: 6;
the heavy chain variable region of the antibody has an amino acid sequence set forth in SEQ ID NO: 11, and the light chain variable region of the antibody has an amino acid sequence set forth in SEQ ID NO: 19;
the heavy chain variable region of the antibody has an amino acid sequence set forth in SEQ ID NO: 17, and the light chain variable region of the antibody has an amino acid sequence set forth in SEQ ID NO: 19;
the heavy chain variable region of the antibody has an amino acid sequence set forth in SEQ ID NO: 13, and the light chain variable region of the antibody has an amino acid sequence set forth in SEQ ID NO: 21;
the heavy chain variable region of the antibody has an amino acid sequence set forth in SEQ ID NO: 13, and the light chain variable region of the antibody has an amino acid sequence set forth in SEQ ID NO: 23;
the heavy chain variable region of the antibody has an amino acid sequence set forth in SEQ ID NO: 15, and the light chain variable region of the antibody has an amino acid sequence set forth in SEQ ID NO: 21;
the heavy chain variable region of the antibody has an amino acid sequence set forth in SEQ ID NO: 15, and the light chain variable region of the antibody has an amino acid sequence set forth in SEQ ID NO: 23;
the heavy chain variable region of the antibody has an amino acid sequence set forth in SEQ ID NO: 11, and the light chain variable region of the antibody has an amino acid sequence set forth in SEQ ID NO: 25; or
the heavy chain variable region of the antibody has an amino acid sequence set forth in SEQ ID NO: 17, and the light chain variable region of the antibody has an amino acid sequence set forth in SEQ ID NO: 25;
preferably, for the anti-TIGIT antibody or the antigen-binding fragment thereof, the antibody comprises a non-CDR region derived from a species other than murine, for example, from a human antibody;
preferably, for the anti-TIGIT antibody or the antigen-binding fragment thereof, the antibody comprises a heavy chain constant region that is an Ig gamma-1 chain C region (e.g., NCBI ACCESSION: P01857), and a light chain constant region that is an Ig kappa chain C region (e.g., NCBI ACCESSION: P01834);
preferably, the antigen-binding fragment is selected from Fab, Fab', F(ab')2, Fd, Fv, dAb, a complementarity determining region fragment, a single chain antibody, a humanized antibody, a chimeric antibody, and a diabody;
preferably, for the anti-TIGIT antibody or the antigen-binding fragment thereof, the antibody binds to TIGIT-mFc with a K_{D} less than 4E-10 or less than 4E-11; preferably, the K_{D} is measured by a Fortebio molecular interaction instrument;
preferably, for the anti-TIGIT antibody or the antigen-binding fragment thereof, the antibody binds to TIGIT with an EC₅₀ less than 1.5 nM, less than 1.2 nM, or less than 1 nM; preferably, the EC₅₀ is measured by a flow cytometer;
preferably, the anti-TIGIT antibody is a monoclonal antibody, a humanized antibody, a chimeric antibody, or a multispecific antibody (e.g., a bispecific antibody);
preferably, for the anti-TIGIT antibody or the antigen-binding fragment thereof, the antibody is an antibody produced by hybridoma cell line LT019 deposited at China Center for Type Culture Collection (CCTCC) under CCTCC NO. C2020208.

3. The pharmaceutical composition according to claim 1, wherein the heavy chain variable region of the immunoglobulin of the anti-PD-1/anti-VEGFA bispecific antibody has an amino acid sequence selected from SEQ ID NO 31 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto; and the light chain variable region of the immunoglobulin has an amino acid sequence selected from SEQ ID NO 33 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto; and the heavy chain variable region of the single chain antibody has an amino acid sequence selected from SEQ ID NO 41 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto; and the light chain variable region of the single chain antibody has an amino acid sequence selected SEQ ID NO 43 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto;
preferably, in the anti-PD-1/anti-VEGFA bispecific antibody, the first protein functional region is linked to the second protein functional region either directly or via a linker fragment; and/or the heavy chain variable region of the single chain antibody is linked to the light chain variable region of the single chain antibody either directly or via a linker fragment;
preferably, in the anti-PD-1/anti-VEGFA bispecific antibody, the linker fragment is (GGGGS)n, wherein n is a positive integer; preferably, n is 1, 2, 3, 4, 5, or 6;
preferably, in the anti-PD-1/anti-VEGFA bispecific antibody, the numbers of the first protein functional region and the second protein functional region are each independently 1, 2, or more;
preferably, in the anti-PD-1/anti-VEGFA bispecific antibody, the single chain antibody (preferably the heavy chain variable region) is linked to the C terminus of the heavy chain of the immunoglobulin;
wherein, according to the EU numbering system, the immunoglobulin comprises a heavy chain constant region having the following mutations:
L234A and L235A; or
L234A and G237A; or
L235A and G237A; or
L234A, L235A and G237A;
preferably, the anti-PD-1/anti-VEGFA bispecific antibody comprises:
a first protein functional region targeting PD-1, and
a second protein functional region targeting VEGFA;
the number of the first protein functional region is 2, and the number of the second protein functional region is 1;
wherein the first protein functional region is a single chain antibody, wherein the single chain antibodies are identical or different; the second protein functional region is an immunoglobulin;
the heavy chain of the immunoglobulin has an amino acid sequence set forth in SEQ ID NO 31 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto, and the light chain has an amino acid sequence set forth in SEQ ID NO 33 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto;
the heavy chain variable region of the single chain antibody has an amino acid sequence set forth in SEQ ID NO 41 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto, and the light chain variable region of the single chain antibody has an amino acid sequence set forth in SEQ ID NO 43 or a sequence having at least 60%, 70%, 80%, 81%, 82%, 83%, 84%, 85%, 86%, 87%, 88%, 89%, 90%, 91%, 92%, 93%, 94%, 95%, 96%, 97%, 98%, or 99% homology thereto;
the single chain antibody is linked to the C terminus of the heavy chain of the immunoglobulin;
the first protein functional region is linked to the second protein functional region via a first linker fragment; the heavy chain variable region of the single chain antibody is linked to the light chain variable region of the single chain antibody via a second linker fragment; the first linker fragment and the second linker fragment are identical or different;
preferably, the first linker fragment and the second linker fragment each have an amino acid sequence independently selected from SEQ ID NO: 52 and SEQ ID NO: 53;
preferably, the first linker fragment and second linker fragment both have an amino acid sequence set forth in SEQ ID NO: 53;
preferably, the heavy chain of the anti-PD-1/anti-VEGFA bispecific antibody has an amino acid sequence set forth in SEQ ID NO: 27, the light chain has an amino acid sequence set forth in SEQ ID NO: 29, and the bispecific antibody has a structure of IgG-scFv, wherein the IgG part is an anti-VEGFA antibody, and the scFv part is an anti-PD-1 antibody.

4. The pharmaceutical composition according to any of claims 1-3, wherein the anti-TIGIT antibody or the antigen-binding fragment thereof and the anti-PD-1/anti-VEGFA bispecific antibody or the antigen-binding fragment thereof are present in a mass ratio, on antibody basis, of 1:5-5:1, for example, 1:5, 1:4, 1:3, 1: 2, 1:1, 2:1, 3:1, 4: 1, or 5: 1.

5. A kit comprising a first product and a second product in separate packages, wherein,
the first product comprises the anti-TIGIT antibody or the antigen-binding fragment thereof as defined in any of claims 1-4;
the second product comprises the anti-PD-1/anti-VEGFA bispecific antibody or the antigen-binding fragment thereof as defined in any of claims 1-4;
preferably, the kit further comprises a third product in a separate package comprising one or more chemotherapeutics,
preferably, the first product and the second product further independently comprise one or more pharmaceutically acceptable excipients;
preferably, the combination product further comprises a package insert;
preferably, in the kit, the anti-TIGIT antibody or the antigen-binding fragment thereof and the anti-PD-1/anti-VEGFA bispecific antibody or the antigen-binding fragment thereof are present in a mass ratio, on antibody basis, of 1:5-5:1, for example, 1:5, 1:4, 1:3, 1:2, 1:1, 2:1, 3:1, 4:1, or 5:1.

6. A method for treating and/or preventing a tumor, comprising: administering to a subject in need an effective amount of the anti-TIGIT antibody or the antigen-binding fragment thereof as defined in any of claims 1-4 and/or the anti-PD-1/anti-VEGFA bispecific antibody or the antigen-binding fragment thereof as defined in any of claims 1-4;
preferably, one or more chemotherapeutics or therapeutic procedures are administered in combination (preferably the chemotherapeutic is a chemotherapeutic or a growth inhibitor (e.g., an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxin or an anti-tumor antibiotic, a proteasome inhibitor, an anti-microtubule agent, an EGFR antagonist, a retinoid, a tyrosine kinase inhibitor, a histone deacetylase inhibitor, and a combination thereof), a targeted therapeutic (e.g., a B-raf inhibitor, an MEK inhibitor, a K-ras inhibitor, a c-Met inhibitor, an Alk inhibitor, a phosphatidylinositol 3-kinase inhibitor, an Akt inhibitor, an mTOR inhibitor, a diphosphatidylpinosite 3-kinase/mTOR inhibitor, and a combination thereof), an antibody-drug conjugate (such as maytansine, monomethyl auristatin E, calicheamicin, esperamicin, and a radioisotope chelator), an antimetabolite, an antibiotic, a botanical drug, and/or a hormonal drug, preferably cyclophosphamide, pemetrexed, a platinum-based drug such as cisplatin, carboplatin and oxaliplatin, adriamycin, paclitaxel, a vinca alkaloid, tamoxifen, megestrol, goserelin, asparaginase, and/or a fluorouracil antineoplastic),
preferably, the anti-TIGIT antibody, the anti-PD-1/anti-VEGFA bispecific antibody, and the anti-tumor chemotherapeutic are administered simultaneously or sequentially; more preferably, the anti-TIGIT antibody and the anti-PD-1/anti-VEGFA bispecific antibody are administered before or after a surgical treatment, and/or before or after a radiation therapy;
preferably, the anti-TIGIT antibody, the anti-PD-1/anti-VEGFA bispecific antibody and/or the chemotherapeutic are in a form suitable for intravenous injection or intravenous drip infusion, preferably in a liquid form;
preferably, the chemotherapeutic or the growth inhibitor is selected from an alkylating agent, an anthracycline, an anti-hormonal agent, an aromatase inhibitor, an anti-androgen agent, a protein kinase inhibitor, a lipid kinase inhibitor, an antisense oligonucleotide, a ribozyme, an antimetabolite, a topoisomerase inhibitor, a cytotoxic agent or an anti-tumor antibiotic, a proteasome inhibitor, an anti-microtubule agent, an EGFR antagonist, a retinoid, a tyrosine kinase inhibitor, a histone deacetylase inhibitor, and a combination thereof;
preferably, the targeted therapeutic is selected from a B-raf inhibitor, an MEK inhibitor, a K-ras inhibitor, a c-Met inhibitor, an Alk inhibitor, a phosphatidylinositol 3-kinase inhibitor, an Akt inhibitor, an mTOR inhibitor, a diphosphatidylglycol 3-kinase/mTOR inhibitor, and a combination thereof; preferably, the antibody-drug conjugate comprises a drug selected from the group consisting of: maytansine, monomethyl auristatin E, calicheamicin, esperamicin, and a radioisotope chelating agent;
preferably, the tumor is selected from one or more of the following:
cervical cancer (e.g., metastatic cervical cancer), endometrial cancer, lung cancer such as small cell lung cancer and non-small cell lung cancer (e.g., squamous non-small cell lung cancer or non-squamous non-small cell lung cancer), throat cancer, esophageal cancer, esophageal squamous cancer, thyroid cancer, mesothelioma, gastric cancer (e.g., advanced gastric cancer, gastrointestinal cancer, gastric adenocarcinoma, or gastroesophageal junction adenocarcinoma), liver cancer (e.g., hepatocellular carcinoma), intestinal cancer, rectal cancer, colon cancer, colorectal cancer, cholangiocarcinoma, hepatobiliary cancer, biliary tract cancer, cholangiocarcinoma, pancreatic cancer, pancreatic cancer, renal cancer (e.g., renal cell carcinoma), ovarian cancer (e.g., advanced ovarian cancer), fallopian tube cancer, peritoneal cancer, glioma (e.g., neuroglioma and recurrent glioma), skin cancer, melanoma, leukemia (e.g., acute myeloid leukemia), lymphoma (e.g., Hodgkin's lymphoma and non-Hodgkin's lymphoma), plasma cell cancer, bone cancer, sarcoma, osteosarcoma, chondrosarcoma, neuroblastoma, myeloma (e.g., multiple myeloma), large cell neuroendocrine cancer, urothelial carcinoma (e.g., upper urothelial carcinoma or bladder cancer), prostate cancer, testicular cancer, peripheral T-cell lymphoma, nasopharyngeal cancer, high microsatellite instability (MSI-H) or mismatch repair deficient (dMMR) solid tumors, head and neck cancer, brain cancer (e.g., aggressive brain cancer, such as glioblastoma), squamous cell carcinoma, basal cell carcinoma, adenoma, breast cancer (e.g., triple-negative breast cancer), thymus cancer, ileocecal adenocarcinoma, ampullate adenocarcinoma, mucinous or serous cystadenocarcinoma, leiomyosarcoma, rhabdomyosarcoma, chorioepithelioma, malignant hydatidiform mole, malignant sertoli cell-stromal cell tumor, malignant granulocytoma, dysgerminoma, glioblastoma, mycosis, Merkel cell carcinoma, and other hematologic malignancies,
preferably, the unit dose of the anti-TIGIT antibody and/or the anti-PD-1/anti-VEGFA bispecific antibody as defined in any of claims 1-4 is 0.1-100 mg, preferably 1-10 mg, per kg body weight; alternatively, the unit dose of the anti-TIGIT antibody and/or the anti-PD-1/anti-VEGFA bispecific antibody as defined in any aspect of the present invention is 10-1000 mg, preferably 50-500 mg, 100-400 mg, 150-300 mg, 150-250 mg, or 200 mg, in each subject, preferably, the dose is administered from twice daily to about once every other day, or once every 3 days, 4 days, 5 days, 6 days, 10 days, 1 week, 2 weeks, 3 weeks, 4 weeks, 5 weeks, or 6 weeks;
preferably, the route of administration is intravenous drip infusion or intravenous injection.

7. A unit formulation, preferably used for treating a tumor, comprising: 1-10000 mg (preferably 10-1000 mg, preferably 50-500 mg, 100-400 mg, 150-300 mg, 150-250 mg, or 200 mg) of the anti-TIGIT antibody as defined in any of claims 1-4, 1-10000 mg (preferably 1-1000 mg, preferably 50-500 mg, 100-400 mg, 150-300 mg, 150-250 mg, 200 mg, or 100 mg) of the anti-PD-1/anti-VEGFA bispecific antibody as defined in any of claims 1-4, and optionally one or more of the chemotherapeutics (such as a platinum-based drug and/or a fluorouracil antineoplastic) as defined in claim 6, wherein the anti-TIGIT antibody, the anti-PD-1/anti-VEGFA bispecific antibody and the chemotherapeutic are in separate packages.

8. A single dose unit, preferably used for treating a tumor, comprising: 0.1-10000 mg (preferably 1-1000 mg, preferably 50-500 mg, 100-400 mg, 150-300 mg, 150-250 mg, 200 mg, or 100 mg) of the anti-TIGIT antibody as defined in any of claims 1-4 and 0.1-10000 mg (preferably 1-1000 mg, preferably 50-500 mg, 100-400 mg, 150-300 mg, 150-250 mg, 200 mg, or 100 mg) of the anti-PD-1/anti-VEGFA bispecific antibody as defined in any of claims 1-4.
